(19) Europäisches Patentamt

European Patent Office

Office européen des brevets

(11) **EP 4 277 697 B1**

(12) **EUROPEAN PATENT SPECIFICATION**

(45) Date of publication and mention
of the grant of the patent:
**04.12.2024 Bulletin 2024/49**

(21) Application number: **22703747.0**

(22) Date of filing: **13.01.2022**

(51) International Patent Classification (IPC):
***A61N 1/36*** (2006.01)

(52) Cooperative Patent Classification (CPC):
**A61N 1/36139**

(86) International application number:
**PCT/US2022/012316**

(87) International publication number:
**WO 2022/155339 (21.07.2022 Gazette 2022/29)**

(54) **MULTIMODAL STIMULATION CONTROL BASED ON ECAPS**

MULTIMODALE STIMULATIONSSTEUERUNG AUF BASIS VON CAPS

COMMANDE DE STIMULATION MULTIMODALE BASÉE SUR DES ECAP

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.01.2021 US 202163136984 P**

(43) Date of publication of application:
**22.11.2023 Bulletin 2023/47**

(73) Proprietor: **Medtronic, Inc.
Minneapolis, Minnesota 55432 (US)**

(72) Inventors:
• **DINSMOOR, David A.
Minneapolis, Minnesota 55432 (US)**
• **VALLEJO, Ricardo
Minneapolis, Minnesota 55432 (US)**
• **HAGEMAN, Kristin N.
Minneapolis, Minnesota 55432 (US)**
• **BINK, Hank
Minneapolis, Minnesota 55432 (US)**
• **SKERKER, Abigail Lauren
Minneapolis, Minnesota 55432 (US)**

(74) Representative: **Zimmermann & Partner
Patentanwälte mbB
Postfach 330 920
80069 München (DE)**

(56) References cited:
WO-A1-2019/067059    WO-A1-2019/246579
WO-A1-2019/246582    US-A1- 2020 054 879

EP 4 277 697 B1

**Description**

TECHNICAL FIELD

This disclosure generally relates to electrical stimulation, and more specifically, control of electrical stimulation.

BACKGROUND

Medical devices may be external or implanted and may be used to deliver electrical stimulation to patients via various tissue sites to treat a variety of symptoms or conditions such as chronic pain, tremor, Parkinson's disease, epilepsy, urinary or fecal incontinence, sexual dysfunction, obesity, or gastroparesis. A medical device may deliver electrical stimulation therapy via one or more leads that include electrodes located proximate to target locations associated with the brain, the spinal cord, pelvic nerves, peripheral nerves, or the gastrointestinal tract of a patient. Stimulation proximate the spinal cord, proximate the sacral nerve, within the brain, and proximate peripheral nerves are often referred to as spinal cord stimulation (SCS), sacral neuromodulation (SNM), deep brain stimulation (DBS), and peripheral nerve stimulation (PNS), respectively. Electrical stimulation may be delivered by the medical device as a train of pulses, and the values of the parameters defining the pulses may be altered. Documents WO 2019/246582 A1 and WO 2019/246579 A1 relate to electrical stimulation devices.

SUMMARY

The invention is defined by the appended claims. In general, systems, devices, and techniques are described for managing the delivery of electrical stimulation based ECAP signals. A stimulation system may deliver different pulse trains defined by one or more different parameter values and/or delivered via different electrode combinations. For example, one pulse train may be referred to as a prime pulse train and a second pulse train may be referred to as a base pulse train, where the prime pulse train and the base pulse train work together to provide multimodal stimulation therapy to the patient. Although these different pulse trains may deliver different stimulation pulses to the patient and to different tissues, the different pulse trains may be linked in how they are perceived by the patient or how they combine to provide a perceived effect for the patient.

In some examples, the patient may benefit from the system automatically adjusting the value of one or more stimulation parameters that define the prime pulse train and/or the base pulse train that are a part of the multimodal stimulation therapy. The system may sense an ECAP signal elicited by a pulse of the base pulse train, as the base pulse train may be delivered at a pulse frequency conducive to sensing the elicited ECAP signal. The system may then determine a characteristic value of the ECAP signal and adjust one or more parameters that define subsequent pulses of the prime pulse train and, in some examples, subsequent pulses of the base pulse train. For example, the system may adjust an amplitude of the prime pulse train pulses, an electrode combination used to deliver the subsequent prime pulse train, or any other parameters associated with the prime pulse train, the base pulse train, and/or any other aspect of the multimodal stimulation therapy.

In this manner, the system may automatically adjust the values of the different pulse trains based on evoked compound action potential (ECAP) signals sensed from a patient. When a patient moves, the distance between implanted electrodes and target nerves changes. For example, electrodes implanted along the spinal column are closer to the spinal cord when a subject lies in a supine posture state as compared to a standing posture state. Similarly, the implanted electrodes may move closer to the spinal cord when a subject coughs or sneezes. This changing distance between the electrodes and target tissue affects neural recruitment for a given intensity of delivered stimulation and can cause the patient's perception and/or therapeutic benefit to also change. Therefore, a characteristic value of the ECAP signal can represent the change in distance, and a system can modulate electrical stimulation, such as adjusting a parameter of the prime pulse train and/or the base pulse train of the multimodal stimulation, using the characteristic value as feedback. The system can increase or decrease a parameter of the pulse trains, such as amplitude, in order to maintain a target ECAP value or keep pulse train amplitudes below a threshold ECAP value, as some examples.

In one example, a system includes processing circuitry configured to control delivery of a first train of electrical stimulation pulses at a first frequency to a first target tissue; control delivery of a second train of electrical stimulation pulses at a second frequency to a second target tissue different from the first target tissue, wherein at least some electrical stimulation pulses of the first train of electrical stimulation pulses are interleaved with at least some electrical stimulation pulses of the second train of electrical stimulation pulses, and wherein the first frequency is greater than the second frequency; receive an evoked compound action potential (ECAP) signal elicited by a pulse of the second train of electrical stimulation pulses; adjust, based on the ECAP signal, a first value of a parameter that at least partially defines the first train of electrical stimulation pulses to a second value; and responsive to adjusting the first value of the parameter to the second value, control delivery of subsequent pulses of the first train of electrical stimulation pulses according to the second value of the parameter.

In another example, a method includes controlling, by processing circuitry, delivery of a first train of electrical stimulation pulses at a first frequency to a first target tissue; controlling, by the processing circuitry, delivery of a second train of electrical stimulation pulses at a second frequency to a second target tissue different from the first target tissue, wherein at least some electrical stimulation pulses of the first train of electrical stimulation pulses are interleaved with at least some electrical stimulation pulses of the second train of electrical stimulation pulses, and wherein the first frequency is greater than the second frequency; receiving, by the processing circuitry, an evoked compound action potential (ECAP) signal elicited by a pulse of the second train of electrical stimulation pulses; adjusting, by the processing circuitry and based on the ECAP signal, a first value of a parameter that at least partially defines the first train of electrical stimulation pulses to a second value; and responsive to adjusting the first value of the parameter to the second value, controlling, by the processing circuitry, delivery of subsequent pulses of the first train of electrical stimulation pulses according to the second value of the parameter.

In another example, a computer-readable storage medium including instructions that, when executed by processing circuitry, cause the processing circuitry to control delivery of a first train of electrical stimulation pulses at a first frequency to a first target tissue; control delivery of a second train of electrical stimulation pulses at a second frequency to a second target tissue different from the first target tissue, wherein at least some electrical stimulation pulses of the first train of electrical stimulation pulses are interleaved with at least some electrical stimulation pulses of the second train of electrical stimulation pulses, and wherein the first frequency is greater than the second frequency; receive an evoked compound action potential (ECAP) signal elicited by a pulse of the second train of electrical stimulation pulses; adjust, based on the ECAP signal, a first value of a parameter that at least partially defines the first train of electrical stimulation pulses to a second value; and responsive to adjusting the first value of the parameter to the second value, control delivery of subsequent pulses of the first train of electrical stimulation pulses according to the second value of the parameter.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages of the techniques will be apparent from the description and drawings, and from the claims.

BRIEF DESCRIPTION OF DRAWINGS

FIG. 1 is a conceptual diagram illustrating an example system that includes a medical device programmer and an IMD according to the techniques of the disclosure.

FIG. 2 is a block diagram of the example IMD of FIG. 1.

FIG. 3 is a block diagram of the example external programmer of FIG. 1.

FIG. 4 is a timing diagram illustrating an example of electrical stimulation pulses delivered according to different stimulation patterns.

FIG. 5A is a graph of an example ECAP signal sensed from a stimulation pulse.

FIGS. 5B, 5C, and 5D are timing diagrams illustrating example electrical stimulation pulses and respective sensed ECAPs, in accordance with one or more techniques of this disclosure.

FIG. 6 is a flowchart illustrating an example operation for delivering control stimulation pulses and informed stimulation pulses, in accordance with one or more techniques of this disclosure.

FIG. 7 is a diagram illustrating an example technique for adjusting electrical stimulation therapy.

FIG. 8 is a flowchart illustrating an example operation for controlling stimulation therapy as described in FIG. 7.

Like reference characters denote like elements throughout the description and figures.

DETAILED DESCRIPTION

The disclosure describes examples of medical devices, systems, and techniques for adjusting electrical stimulation based on ECAP signals. Electrical stimulation therapy is typically delivered to a target tissue (e.g., nerves of the spinal cord or muscle) of a patient via two or more electrodes. Parameters of the electrical stimulation therapy (e.g., electrode combination, voltage or current amplitude, pulse width, pulse frequency, etc.) are selected by a clinician and/or the patient to provide relief from various symptoms, such as pain, nervous system disorders, muscle disorders, etc.

In some cases, a medical device can provide therapy to a patient (e.g., pain relief therapy) by using multimodal stimulation (e.g., differential targeted multiplexed stimulation). Multiple modal stimulation includes different pulse trains (e.g., a prime pulse train and a base pulse train) defined by different stimulation parameters such as different frequencies and different electrode combinations. Since different types of cells, such as glial cells and neurons, respond differently to electrical fields, it is then possible to differentially modulate the response of these cell populations with distinctly different electrical parameters. For example, the prime pulse train can be delivered to affect glial cells and the base pulse train can be delivered to affect neurons. Generally, the prime pulse train is delivered at a higher pulse frequency than the base pulse train, as described herein.

However, as the patient moves, the distance between the electrodes and the target tissues can change. Since neural recruitment at the nerves is a function of stimulation intensity (e.g., amplitude and/or pulse frequency) and distance between the target tissue and the electrodes, movement of the electrode closer to the target tissue may result in increased neural recruitment (e.g., possible painful sensations or adverse motor function), and movement of the electrode further from the target tissue may result in decreased efficacy of the therapy for the patient. Certain patient postures (which may or may not include patient activity) may be representative of respective distances (or changes in distance) between electrodes and nerves and thus be an informative feedback variable for modulating stimulation therapy.

in some examples, a patient may experience discomfort or pain caused by transient patient conditions, which is referred to herein as transient overstimulation. The electrodes can move closer to the target tissue for a number of reasons including coughing, sneezing, laughing, valsalva maneuvers, leg lifting, cervical motions, deep breathing, or another transient patient movement. If a system is delivering stimulation during these movements, the patient may perceive the stimulation as stronger (and possibly uncomfortable) due to the decreased distance between electrodes and target tissue in a short amount of time. Although a patient may anticipate such movements and preemptively reduce stimulation intensity in an attempt to avoid these uncomfortable sensations, these patient actions interfere with normal activities and may not be sufficient to avoid uncomfortable stimulation at all times.

ECAPs are a measure of neural recruitment because each ECAP signal represents the superposition of electrical potentials generated from a population of axons firing in response to an electrical stimulus (e.g., a stimulation pulse). Changes in a characteristic (e.g., an amplitude of a portion of the signal or area under the curve of the signal) of an ECAP signals occur as a function of how many axons have been activated by the delivered stimulation pulse. For a given set of parameter values that define the stimulation pulse and a given distance between the electrodes and target nerve, the detected ECAP signal may have a certain characteristic value (e.g., amplitude, or area under a curve). Therefore, a system can determine that the distance between electrodes and nerves has increased or decreased in response to determining that the measured ECAP characteristic value has increased or decreased. For example, if the set of parameter values stays the same and the ECAP characteristic value of amplitude increases, the system can determine that the distance between electrodes and the nerve has decreased.

In some examples, effective stimulation therapy, such as multimodal therapy, may rely on a certain level of neural recruitment at a target nerve. This effective stimulation therapy may provide relief from one or more conditions (e.g., patient perceived pain) without an unacceptable level of side effects (e.g., overwhelming perception of stimulation). However, if the patient changes posture or otherwise engages in physical activity, the distance between the electrodes and the nerve changes as well. This change in distance can cause loss of effective therapy and/or side effects if the parameter values that define stimulation are not adjusted to compensate for the change in distance. Moreover, the different distance between electrodes and the target nerve (e.g., caused by a shift from one posture state to another) may also result in different sensitivities to stimulation intensity (e.g., smaller distances may result in greater sensitivities to changes in stimulation intensity). If a system does not adjust the control policy for these changes, adjustments to stimulation parameter values may not be sufficient to maintain effective therapy or may provide stimulation that is too strong at that posture state. Therefore, it may be beneficial to maintain effective therapy by the system adjusting how stimulation intensity is changed within a given posture state and/or changing target ECAP characteristic values when a posture state of the patient has changed. Moreover, if a user adjusts a parameter value of one pulse train of therapy, the user may also need to adjust a parameter value of a second pulse train. In situations in which the system delivers control pulses and informed pulses, the user may not even have access to adjusting amplitude of the control pulses, for example, It may be difficult for a system to employ ECAP sensing into higher frequency pulse trains or other stimulation paradigms that incorporate different areas of stimulation.

As described herein, systems, devices, and techniques provide solutions to one or more of the above-referenced problems by adjusting one or more parameters that define multimodal stimulation based on sensed ECAP signals elicited from respective pulses of one pulse train of the multimodal stimulation. Pulses that elicit detectable ECAP signals are described as control pulses, and pulses that can be adjusted (e.g., adjusting a value of one or more parameters that at least partially define these pulses) are referred to as informed pulses because they are informed by the ECAP elicited by the control pulses. In this manner, although an informed pulse may elicit an ECAP, the system does not sense or use the ECAP elicited by an informed pulse to adjust any aspect of the stimulation.

For example, the base pulse train may be delivered at a lower frequency than the prime pulse train such that the system can sense an ECAP elicited from a pulse of the base pulse train. In this manner, the base pulse train may be referred to a type of control pulses and the prime pulse train (or multiple prime pulse trains) may be referred to as a type of informed pulses herein. Electrical stimulation may be generally delivered to a patient by the medical device in a train of stimulation pulses, and parameters that define the stimulation pulses may include pulse amplitude (current and/or voltage), pulse frequency, pulse width, pulse shape, and/or electrode combination. The system may alter, adjust, change, or otherwise modulate one or more parameters of the stimulation pulses over time in order to maintain a desired level of stimulation efficacy for the patient. For example, the system may adjust or modulate the value of a stimulation parameter that at least partially defines subsequent pulses of the prime pulse train, such as an amplitude, an electrode combination, a duty cycle, an interpulse interval, etc. In some examples, the system may also adjust as aspect of the closed loop stimulation based on the ECAP characteristic value, such as a gain value that affects the response of the system to changes in the ECAP characteristic value. The system may determine the ECAP characteristic value from one or more aspects of the ECAP signal representative of changes in stimulation.

Nerve impulses detectable as the ECAP signal travel quickly along the nerve fiber after the delivered stimulation pulse first depolarizes the nerve. If the stimulation pulse that elicits the ECAP signal is delivered by first electrodes has a pulse width that is too long (or another pulse is delivered too quickly), different electrodes configured to sense the ECAP will sense a stimulation pulse itself as an artifact that obscures the lower amplitude ECAP signal. Although sensing electrodes could be positioned farther away from where the stimulation pulse is delivered to avoid this artifact, the ECAP signal loses fidelity as the electrical potentials propagate from the electrical stimulus because different nerve fibers propagate electrical potentials at different speeds. Therefore, sensing the ECAP at a far distance from the stimulating electrodes may avoid the artifact caused by a stimulation pulse with a long pulse width, but the ECAP signal may lose fidelity needed to detect changes to the ECAP signal that occur when the electrode to target tissue distance changes. In other words, the system may not be able to identify, at any distance from the stimulation electrodes, ECAPs elicited by certain stimulation pulses having relatively long pulse widths that interfere with detection of ECAP signals (e.g., stimulation pulses that may be configured to provide a therapeutic effect for the patient).

To avoid this ECAP detection problem with some stimulation pulses, a medical device may be configured to deliver a plurality of control pulses (e.g., at least some pulses of the base pulse train) and a plurality of informed pulses (e.g., at least one pulses of the prime pulse train(s)) in some examples. Informed pulses may be configured to contribute to a therapeutic effect for the patient, but the informed pulses may have a stimulation parameter, such as a pulse width or high pulse frequency, that causes at least a portion of the pulse to overlaps with the ECAP signal and prevents the system from detecting the ECAP signal or otherwise using the ECAP signal for direct feedback for modulating parameter values of the informed pulses. The plurality of control pulses, on the other hand, may be configured to elicit detectable ECAP signals, and in the case of a base pulse train, configured to contribute to effective stimulation therapy along with the informed pulses. For example, the control pulses may have a pulse width that is short enough, and a pulse frequency low enough, to avoid interfering with the ECAP signal detection. The control pulses may or may not contribute to a therapeutic effect for the patient. In this manner, the system may be configured to adjust one or more parameters that define the informed pulses (e.g., the prime pulse train(s)) based on the detectable ECAP signals elicited by one or more control pulses (e.g., the base pulse train(s)).

In one example described herein, a medical device can deliver a plurality of informed pulses to provide a therapy to the patient and a plurality of control pulses. The control pulses may be interleaved with the delivery of the informed pulses, as is the case with the prime pulse train(s) and base pulse train(s) of multimodal stimulation. For example, the medical device may alternate the delivery of informed pulses with control pulses such that a control pulse is delivered, and an ECAP signal is sensed, between consecutive informed pulses. In some cases, depending on innervation and distance between the areas affected by the control pulses and the informed pulses, one or more informed pulses may partially or completely overlap in time such that an ECAP signal elicited by a control pulse is unaffected by the delivery of an informed pulse over at least some of the same time. In some examples, multiple control pulses are delivered, and respective ECAP signals sensed, between the delivery of consecutive informed pulses. In some examples, multiple informed pulses will be delivered between consecutive control pulses. In any case, the informed pulses may be delivered according to a predetermined pulse frequency selected so that the informed pulses can produce a therapeutic result for the patient. One or more control pulses are then delivered, and the respective ECAP signals sensed, within one or more time windows between consecutive informed pulses delivered according to the predetermined pulse frequency. In this manner, a medical device can administer informed pulses from the medical device uninterrupted while ECAPs are sensed from control pulses delivered during times at which the informed pulses are not being delivered. In other examples described herein, ECAPs are sensed by the medical device in response to the informed pulses delivered by the medical device, and control pulses are not used to elicit ECAPs.

The system may monitor one or more characteristic values that represent detected ECAP signals and adjust a stimulation parameter value in an attempt to achieve a target ECAP characteristic value or avoid a threshold ECAP value. The system may adjust an informed parameter that at least partially defines subsequent informed pulses and may adjust a control parameter that at least partially defines subsequent control pulses. When adjusting the informed parameter value and/or the control parameter value in response to determining that the sensed characteristic value of the ECAP signal is below or above the target ECAP characteristic value, the system may employ a gain value that represents the magnitude, or rate, of change applied to a stimulation parameter in order to achieve the target ECAP characteristic value. The gain value may be the same or different for informed pulses and control pulses. In some examples, the system may apply a scaling factor or otherwise adjust the gain value so that it is appropriate for informed pulses and control pulses that may have different amplitudes or other parameters. For example, if the control pulse has a higher amplitude value than the informed pulse, the system may effectively reduce the gain value, or reduce the effect of the gain value, on the change to the informed pulse amplitude because the lower amplitude value of the informed pulse may not need to be changes as much as the control pulse amplitude. The system can thus increase or decrease a stimulation parameter according to the gain value in order to maintain the target ECAP characteristic value.

In some examples, the gain value may be a multiplier applied to a difference between a target ECAP characteristic value and a detected ECAP characteristic value. If the gain value is constant, the result is a stimulation parameter value that changes linearly. For example, the system may select one gain value for any detected ECAP characteristic value that is less than the target ECAP characteristic value, and the system may select a different gain value for any detected ECAP characteristic value that is greater than the target ECAP characteristic value. In other examples, the gain value may be calculated using a function that may be linear or non-linear. Put another way, for a given input or set of inputs (e.g., the detected ECAP characteristic value and/or posture state may be an input that affects the calculated gain value) the system may calculate different gain values for increasing stimulation intensity and/or decreasing stimulation intensity.

In one example, the system may determine a gain value that changes for different sensed ECAP characteristic values or different differences between the sensed ECAP characteristic value and a target ECAP characteristic value. A changing gain value (via a linear or non-linear function) would result in a non-linear function that determines the adjusted stimulation parameter (e.g., the output of the non-linear function). For example, the system may adjust the stimulation parameter value exponentially or logarithmically according to the difference between the sensed ECAP characteristic value and the threshold ECAP amplitude. In one example, the gain value is calculated by multiplying the difference between the sensed ECAP characteristic value and the threshold ECAP amplitude to a multiplier (e.g., a linear function) such that the gain value changes according to that difference between the sensed ECAP characteristic value and the threshold ECAP amplitude. In some examples, the gain value may represent a value selected from a table that stores gain values for respective difference values between the sensed ECAP characteristic value and the threshold ECAP amplitude. The table may result in a linear or non-linear function for determining the next stimulation parameter value.

In another type of control policy (e.g., type of closed-loop feedback scheme), the system may employ a threshold ECAP characteristic value instead of a target ECAP characteristic value. The system may monitor characteristic values for sensed ECAI' signals and reduce one or more stimulation parameter values (e.g., informed parameter values and/or control parameter values) from a predetermined value only in response to the characteristic value exceeding the threshold ECAP characteristic value. In other words, the system may be configured to attempt to keep characteristic values of sensed ECAP signals below the threshold ECAP characteristic value and only increase the stimulation parameter back up to the predetermined value in response to the characteristic value dropping back below the threshold ECAP characteristic value.

Informed pulses and control pulses are generally described herein as different stimulation pulses reflective of different types of electrical stimulation, such as prime pulse trains and base pulse trains, respectively. The control pulses may be at least partially interleaved with at least some of the informed pulses. For example, the system may alternate delivery of one first pulse with delivery of one second pulse. In another example, the number of first pulses may differ from the number of second pulses by a ratio or percentage. The ratio could be 1:1 when the first and second pulses are fully interleaved. The ratio could be 10:1 first pulses to second pulses in examples in which the second pulses are delivered less frequently than the first pulses. In other examples, the ratio could be 1:4 first pulses to second pulses when the second pulses, and respective sensed ECAP signals) occur more frequently than the first pulses.

Although electrical stimulation is generally described herein in the form of electrical stimulation pulses, electrical stimulation may be delivered in non-pulse form in other examples. For example, electrical stimulation may be delivered as a signal having various waveform shapes, frequencies, and amplitudes. Therefore, electrical stimulation in the form of a non-pulse signal may be a continuous signal than may have a sinusoidal waveform or other continuous waveform.

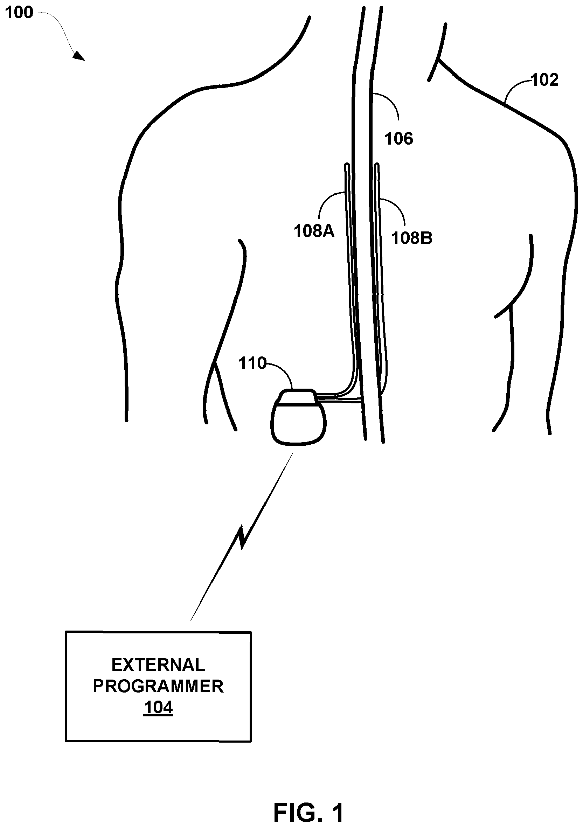

FIG. 1 is a conceptual diagram illustrating example system 100 that includes implantable medical device (IMD) 110 to deliver electrical stimulation therapy to patient 102. Although the techniques described in this disclosure are generally applicable to a variety of medical devices including external devices and IMDs, application of such techniques to IMDs and, more particularly, implantable electrical stimulators (e.g., neurostimulators) will be described for purposes of illustration. More particularly, the disclosure will refer to an implantable SCS system for purposes of illustration, but without limitation as to other types of medical devices or other therapeutic applications of medical devices.

As shown in FIG. 1, system 100 includes an IMD 110, leads 108A and 108B, and external programmer 104 shown in conjunction with a patient 102, who is ordinarily a human patient. In the example of FIG. 1, IMD 110 is an implantable electrical stimulator that is configured to generate and deliver electrical stimulation therapy to patient 102 via one or more electrodes of electrodes of leads 108A and/or 108B (collectively, "leads 108"), e.g., for relief of chronic pain or other symptoms. In other examples, IMD 110 may be coupled to a single lead carrying multiple electrodes or more than two leads each carrying multiple electrodes. In some examples, the stimulation signals, or pulses (e.g., control pulses), may be configured to elicit detectable ECAP signals that IMD 110 may use to determine the posture state occupied by patient 102 and/or determine how to adjust one or more parameters that define stimulation therapy. IMD 110 may be a chronic electrical stimulator that remains implanted within patient 102 for weeks, months, or even years. In other examples, IMD 110 may be a temporary, or trial, stimulator used to screen or evaluate the efficacy of electrical stimulation for chronic therapy. In one example, IMD 110 is implanted within patient 102, while in another example, IMD 110 is an external device coupled to percutaneously implanted leads. In some examples, IMD 110 uses one or more leads, while in other examples, IMD 110 is leadless.

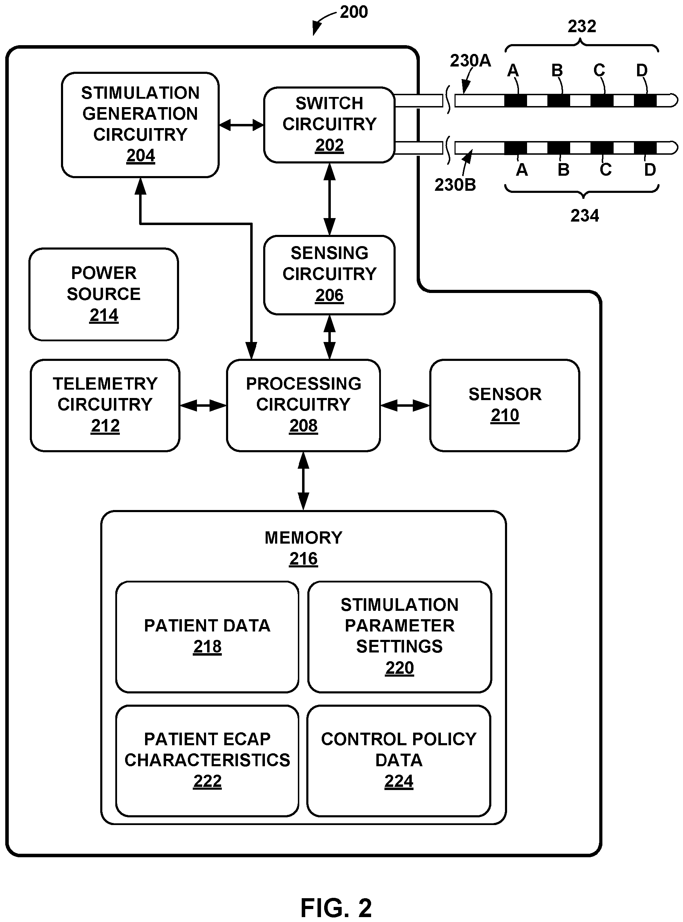

IMD 110 may be constructed of any polymer, metal, or composite material sufficient to house the components of IMD 110 (e.g., components illustrated in FIG. 2) within patient 102. In this example, IMD 110 may be constructed with a biocompatible housing, such as titanium or stainless steel, or a polymeric material such as silicone, polyurethane, or a liquid crystal polymer, and surgically implanted at a site in patient 102 near the pelvis, abdomen, or buttocks. In other examples, IMD 110 may be implanted within other suitable sites within patient 102, which may depend, for example, on the target site within patient 102 for the delivery of electrical stimulation therapy. The outer housing of IMD 110 may be configured to provide a hermetic seal for components, such as a rechargeable or non-rechargeable power source. In addition, in some examples, the outer housing of IMD 110 is selected from a material that facilitates receiving energy to charge the rechargeable power source.

Electrical stimulation energy, which may be constant current or constant voltage-based pulses, for example, is delivered from IMD 110 to one or more target tissue sites of patient 102 via one or more electrodes (not shown) of implantable leads 108. In the example of FIG. 1, leads 108 carry electrodes that are placed adjacent to the target tissue of spinal cord 106. One or more of the electrodes may be disposed at a distal tip of a lead 108 and/or at other positions at intermediate points along the lead. Leads 108 may be implanted and coupled to IMD 110. The electrodes may transfer electrical stimulation generated by an electrical stimulation generator in IMD 110 to tissue of patient 102. Although leads 108 may each be a single lead, lead 108 may include a lead extension or other segments that may aid in implantation or positioning of lead 108. In some other examples, IMD 110 may be a leadless stimulator with one or more arrays of electrodes arranged on a housing of the stimulator rather than leads that extend from the housing. In addition, in some other examples, system 100 may include one lead or more than two leads, each coupled to IMD 110 and directed to similar or different target tissue sites.

The electrodes of leads 108 may be electrode pads on a paddle lead, circular (e.g., ring) electrodes surrounding the body of the lead, conformable electrodes, cuff electrodes, segmented electrodes (e.g., electrodes disposed at different circumferential positions around the lead instead of a continuous ring electrode), any combination thereof (e.g., ring electrodes and segmented electrodes) or any other type of electrodes capable of forming unipolar, bipolar or multipolar electrode combinations for therapy. Ring electrodes arranged at different axial positions at the distal ends of lead 108 will be described for purposes of illustration.

The deployment of electrodes via leads 108 is described for purposes of illustration, but arrays of electrodes may be deployed in different ways. For example, a housing associated with a leadless stimulator may carry arrays of electrodes, e.g., rows and/or columns (or other patterns), to which shifting operations may be applied. Such electrodes may be arranged as surface electrodes, ring electrodes, or protrusions. As a further alternative, electrode arrays may be formed by rows and/or columns of electrodes on one or more paddle leads. In some examples, electrode arrays include electrode segments, which are arranged at respective positions around a periphery of a lead, e.g., arranged in the form of one or more segmented rings around a circumference of a cylindrical lead. In other examples, one or more of leads 108 are linear leads having 8 ring electrodes along the axial length of the lead. In another example, the electrodes are segmented rings arranged in a linear fashion along the axial length of the lead and at the periphery of the lead.

The stimulation parameter set of a stimulation program that defines the stimulation pulses of electrical stimulation therapy by IMD 110 through the electrodes of leads 108 may include information identifying which electrodes have been selected for delivery of stimulation according to a stimulation program, the polarities of the selected electrodes, i.e., the electrode combination for the program, voltage or current amplitude, pulse frequency, pulse width, pulse shape of stimulation delivered by the electrodes. These stimulation parameters values that make up the stimulation parameter set that defines pulses may be predetermined parameter values defined by a user and/or automatically determined by system 1 00 based on one or more factors or user input. Informed pulses may be defined by a set of informed stimulation parameter values and control pulses may be defined by a set of control stimulation parameter values.

Although FIG. 1 is directed to SCS therapy, e.g., used to treat pain, in other examples system 100 may be configured to treat any other condition that may benefit from electrical stimulation therapy. As described herein, system 100 may be configured to provide multimodal stimulation using prime stimulation and base stimulation together. In some examples, system 100 may be used to treat tremor, Parkinson's disease, epilepsy, a pelvic floor disorder (e.g., urinary incontinence or other bladder dysfunction, fecal incontinence, pelvic pain, bowel dysfunction, or sexual dysfunction), obesity, gastroparesis, or psychiatric disorders (e.g., depression, mania, obsessive compulsive disorder, anxiety disorders, and the like). In this manner, system 100 may be configured to provide therapy taking the form of deep brain stimulation (DBS), peripheral nerve stimulation (PNS), peripheral nerve field stimulation (PNFS), cortical stimulation (CS), pelvic floor stimulation, gastrointestinal stimulation, or any other stimulation therapy capable of treating a condition of patient 102.

In some examples, lead 108 includes one or more sensors configured to allow IMD 110 to monitor one or more parameters of patient 102, such as patient activity, pressure, temperature, or other characteristics. The one or more sensors may be provided in addition to, or in place of, therapy delivery by lead 108.

IMD 110 is generally configured to deliver electrical stimulation therapy (e.g., informed pulses and/or control pulses in the form of a prime pulse train and base pulse train, respectively) to patient 102 via selected combinations of electrodes carried by one or both of leads 108, alone or in combination with an electrode carried by or defined by an outer housing of IMD 110. The target tissue for the electrical stimulation therapy may be any tissue affected by electrical stimulation, which may be in the form of electrical stimulation pulses or continuous waveforms. In some examples, the target tissue includes nerves, smooth muscle or skeletal muscle. In the example illustrated by FIG. 1, the target tissue is tissue proximate spinal cord 106, such as within an intrathecal space or epidural space of spinal cord 106, or, in some examples, adjacent nerves that branch off spinal cord 106. Leads 108 may be introduced into spinal cord 106 in via any suitable region, such as the thoracic, cervical or lumbar regions. Stimulation of spinal cord 106 may, for example, prevent pain signals from traveling through spinal cord 106 and to the brain of patient 102. Patient 102 may perceive the interruption of pain signals as a reduction in pain and, therefore, efficacious therapy results. In other examples, stimulation of spinal cord 106 may produce paresthesia which may be reduce the perception of pain by patient 102, and thus, provide efficacious therapy results. In some examples, stimulation of spinal cord 106 or other anatomical structures associated with the spinal cord (e.g., nerves and cells associated with the nervous system) may provide relief from symptoms that may not produce paresthesia. For example, IMD 110 may deliver stimulation with intensities (e.g., values of amplitude and/or pulse width) below a sensory or perception threshold (e.g., sub-threshold stimulation) that reduces pain without paresthesia. In multimodal stimulation, for example, IMD 110 may deliver one pulse train at a higher frequency via one electrode combination and a second pulse train on an interleaved basis with a lower frequency via a second electrode combination, where both pulse trains are delivered at a sub-threshold intensity.

IMD 110 is configured to generate and deliver electrical stimulation therapy to a target stimulation site within patient 102 via the electrodes of leads 108 to patient 102 according to one or more therapy stimulation programs. A therapy stimulation program may generally define informed pulses, but may also define control pulses if the control pulses also contribute to a therapeutic effect). A therapy stimulation program defines values for one or more parameters (e.g., a parameter set) that define an aspect of the therapy delivered by IMD 110 according to that program. For example, a therapy stimulation program that controls delivery of stimulation by IMD 110 in the form of pulses may define values for voltage or current pulse amplitude, pulse width, pulse rate (e.g., pulse frequency), electrode combination, pulse shape, etc. for stimulation pulses delivered by IMD 110 according to that program. In some examples, one or more therapy stimulation programs define multiple different pulse trains that have different parameter values (e.g., different pulse frequencies, amplitudes, pulse widths, and/or electrode combinations) but are delivered on an interleaved basis to together provide a therapy for the patient.

A user, such as a clinician or patient 102, may interact with a user interface of an external programmer 104 to program IMD 110. Programming of IMD 110 may refer generally to the generation and transfer of commands, programs, or other information to control the operation of IMD 110. In this manner, IMD 110 may receive the transferred commands and programs from external programmer 104 to control stimulation, such as stimulation pulses that provide electrical stimulation therapy. For example, external programmer 104 may transmit therapy stimulation programs, stimulation parameter adjustments, therapy stimulation program selections, posture states, user input, or other information to control the operation of IMD 1 10, e.g., by wireless telemetry or wired connection.

In some cases, external programmer 104 may be characterized as a physician or clinician programmer if it is primarily intended for use by a physician or clinician. In other cases, external programmer 104 may be characterized as a patient programmer if it is primarily intended for use by a patient. A patient programmer may be generally accessible to patient 102 and, in many cases, may be a portable device that may accompany patient 102 throughout the patient's daily routine. For example, a patient programmer may receive input from patient 102 when the patient wishes to terminate or change electrical stimulation therapy, or when a patient perceives stimulation being delivered. In general, a physician or clinician programmer may support selection and generation of programs by a clinician for use by IMD 110, whereas a patient programmer may support adjustment and selection of such programs by a patient during ordinary use. In other examples, external programmer 104 may include, or be part of, an external charging device that recharges a power source of IMD 110. In this manner, a user may program and charge IMD 110 using one device, or multiple devices.

As described herein, information may be transmitted between external programmer 104 and IMD 110. Therefore, IMD 110 and external programmer 104 may communicate via wireless communication using any techniques known in the art. Examples of communication techniques may include, for example, radiofrequency (RF) telemetry and inductive coupling, but other techniques are also contemplated. In some examples, external programmer 104 includes a communication head that may be placed proximate to the patient's body near the IMD 110 implant site to improve the quality or security of communication between IMD 110 and external programmer 104. Communication between external programmer 104 and IMD 110 may occur during power transmission or separate from power transmission.

In some examples, IMD 110, in response to commands from external programmer 104, delivers electrical stimulation therapy (e.g., informed pulses and/or control pulses) according to a plurality of therapy stimulation programs to a target tissue site of the spinal cord 106 of patient 102 via electrodes (not depicted) on leads 108. In some examples, IMD 1 10 modifies therapy stimulation programs as therapy needs of patient 102 evolve over time. For example, the modification of the therapy stimulation programs may cause the adjustment of at least one parameter of the plurality of stimulation pulses. When patient 102 receives the same therapy for an extended period, the efficacy of the therapy may be reduced. In some cases, parameters of the plurality of stimulation pulses may be automatically updated.

Efficacy of electrical stimulation therapy may be indicated by one or more characteristics (e.g. an amplitude of or between one or more peaks or an area under the curve of one or more peaks) of an action potential that is evoked by a control pulse delivered by IMD 110 (i.e., a characteristic value of the ECAP signal). Electrical stimulation therapy delivery by leads 108 of IMD 110 may cause neurons within the target tissue to evoke a compound action potential that travels up and down the target tissue, eventually arriving at sensing electrodes of IMD 110. Furthermore, stimulation may also elicit at least one ECAP signal, and ECAPs responsive to stimulation may also be a surrogate for the effectiveness of the therapy. The amount of action potentials (e.g., number of neurons propagating action potential signals) that are evoked may be based on the various parameters of electrical stimulation pulses such as amplitude, pulse width, frequency, pulse shape (e.g., slew rate at the beginning and/or end of the pulse), etc. The slew rate may define the rate of change of the voltage and/or current amplitude of the control pulse at the beginning and/or end of each control pulse or each phase within the pulse. For example, a very high slew rate indicates a steep or even near vertical edge of the pulse, and a low slew rate indicates a longer ramp up (or ramp down) in the amplitude of the control pulse. In some examples, these parameters contribute to an intensity of the electrical stimulation. In addition, a characteristic of the ECAP signal (e.g., an amplitude) may change based on the distance between the stimulation electrodes and the nerves subject to the electrical field produced by the delivered control pulses.

Some example techniques for adjusting stimulation parameter values for stimulation pulses (e.g., informed pulses and/or control pulses that may or may not contribute to therapy for the patient) are based on comparing the value of a characteristic of a measured ECAP signal to a target ECAP characteristic value. In response to delivering a control pulse defined by a set of stimulation parameter values, IMD 110, via two or more electrodes interposed on leads 108, senses electrical potentials of tissue of the spinal cord 106 of patient 102 to measure the electrical activity of the tissue. IMD 110 senses ECAPs from the target tissue of patient 102, e.g., with electrodes on one or more leads 108 and associated sense circuitry. In some examples, IMD 110 receives a signal indicative of the ECAP from one or more sensors, e.g., one or more electrodes and circuitry, internal or external to patient 102. Such an example signal may include a signal indicating an ECAP of the tissue of patient 102. Examples of the one or more sensors include one or more sensors configured to measure a compound action potential of patient 102, or a physiological effect indicative of a compound action potential. For example, to measure a physiological effect of a compound action potential, the one or more sensors may be an accelerometer, a pressure sensor, a bending sensor, a sensor configured to detect a posture of patient 102, or a sensor configured to detect a respiratory function of patient 102. However, in other examples, external programmer 104 receives a signal indicating a compound action potential in the target tissue of patient 102 and transmits a notification to IMD 110.

In the example of FIG. 1, IMD 110 is described as performing a plurality of processing and computing functions. However, external programmer 104 instead may perform one, several, or all of these functions. In this alternative example, IMD 110 functions to relay sensed signals to external programmer 104 for analysis, and external programmer 104 transmits instructions to IMD 110 to adjust the one or more parameters defining the electrical stimulation therapy based on analysis of the sensed signals. For example, IMD 110 may relay the sensed signal indicative of an ECAP to external programmer 104. External programmer 104 may compare the parameter value of the ECAP to the target ECAP characteristic value, and in response to the comparison, external programmer 104 may instruct IMD 110 to adjust one or more stimulation parameter that defines the electrical stimulation informed pulses and, in some examples, control pulses, delivered to patient 102.

In some examples, the system changes the target ECAP characteristic value and/or growth rate(s) over a period of time, such as according to a change to a stimulation threshold (e.g., a perception threshold or detection threshold specific for the patient). The system may be programmed to change the target ECAP characteristic in order to adjust the intensity of informed pulses to provide varying sensations to the patient (e.g., increase or decrease the volume of neural activation). Although the system may change the target ECAP characteristic value, received ECAP signals may still be used by the system to adjust one or more parameter values of the informed pulses and/or control pulses in order to meet the target ECAP characteristic value.

One or more devices within system 100, such as IMD 110 and/or external programmer 104, may perform various functions as described herein. For example, IMD 110 may include stimulation circuitry configured to deliver electrical stimulation, sensing circuitry configured to sense a plurality ECAP signals, and processing circuitry. The processing circuitry may be configured to control the stimulation circuitry to deliver a plurality of electrical stimulation pulses (e.g., control pulses) having different amplitude values and control the sensing circuitry to detect, after delivery of each electrical stimulation pulse of the plurality of electrical stimulation pulses, a respective ECAP signal of the plurality of ECAP signals.

As described herein, IMD 110 may modulate or adjust one or more stimulation parameters that at least partially define electrical stimulation based on a sensed ECAP signals to employ a closed-loop feedback system for adjusting stimulation parameters that define informed pulses and/or control pulses. In one example, IMD 110 includes stimulation generation circuitry configured to generate and deliver electrical stimulation to patient 102 according one or more sets of stimulation parameters (e.g., informed parameters and/or control parameters) that at least partially define the respective informed pulses and/or control pulses of the electrical stimulation. Each set of stimulation parameters may include at least one of an amplitude, a pulse width, a pulse frequency, or a pulse shape.

IMD 110 may also include sensing circuitry configured to sense an ECAP signal elicited by delivered electrical stimulation, such as a control pulse. IMD 110 may also include processing circuitry configured to control delivery of an informed pulses to patient 102 according to a first value of an informed stimulation parameter and determine a characteristic value of the ECAP signal detected from the control pulse. IMD 110 may also receive, from a sensor, a posture state signal representing a posture state of the patient. In some examples, IMD 110 may then determine, based on the posture state signal, a gain value for the stimulation parameter and adjust, based on the characteristic value of the ECAP signal and the gain value, the first value of the informed stimulation parameter to a second value of the informed stimulation parameter. In other examples, IMD 110 may additionally, or alternatively, adjust a target ECAP characteristic value or a threshold ECAP characteristic value based on the posture state signal. IMD 110 may then control subsequent delivery of one or more informed stimulation pulses according to the second value of the informed stimulation parameter. In this manner, an informed parameter value that defines the next informed pulse was "informed" by the ECAP signal elicited by a control pulse.

When IMD 110 is configured to modulate stimulation pulses in order to maintain consistent nerve activation, such as increasing and decreasing a stimulation parameter to maintain a target ECAP characteristic value, IMD 110 may perform an example process. For example, IMD 110 may monitor an amplitude that is the characteristic value of the detected ECAP signal. IMD 110 may adjust the first value to the second value of the informed stimulation parameter by subtracting the amplitude from a target ECAP amplitude value for the patient to generate a differential amplitude. The differential amplitude is the difference between the detected amplitude from the ECAP signal and the target ECAP amplitude value. IMD 110 may then multiply the differential amplitude by the gain value that at least partially defines the control pulses to generate a differential value. The gain value may be a multiplier or fraction. A larger gain value may be associated with posture states at which the distance between electrodes and the target nerve is larger because the distance causes less sensitivity for changes in stimulation pulse intensity. IMD 110 may then add the differential value to a previous amplitude value (e.g., the amplitude value of the last control pulse that was delivered and elicited the ECAP signal) to generate the second value that at least partially defines the next control pulse to be delivered to patient 102. IMD 110 may then multiple the differential value by a scaling factor to generate an informed differential value representing how much the amplitude of the informed pulses needs to change. The scaling factor may be greater than one when the informed pulse amplitude is greater than the control pulse amplitude, and conversely, the scaling factor may be less than one when the informed pulse amplitude is less than the control pulse amplitude. IMD 110 can then add the informed differential value to the previous amplitude value of the informed pulses to generate a second value of the informed pulses for subsequent delivery to the patient.

In other examples, IMD 110 may not attempt to maintain consistent nerve activation by modulating stimulation pulses to achieve a target ECAP characteristic value. Instead, IMD 110 may monitor characteristic values of ECAP signals and only take action when the characteristic value exceeds a threshold ECAP characteristic value. Characteristic values exceeding the threshold ECAP characteristic values may be indicative of increased stimulation perception that may be above an uncomfortable threshold or pain threshold for the patient. Therefore, reducing stimulation pulse intensity when the characteristic value exceeds this level of stimulation may reduce the likelihood that patient 102 experiences any uncomfortable sensations that may occur as a result of posture state changes or any transient movement. For example, IMD 110 may be configured to compare the characteristic value of the ECAP signal to a threshold ECAP characteristic value and determine that the characteristic value of the ECAP signal is greater than the threshold ECAP characteristic value. Responsive to determining that the characteristic value of the ECAP signal is greater than the threshold ECAP characteristic value, IMD 110 may be configured to decrease the first value of the informed stimulation parameter to the second value of the informed stimulation parameter for subsequent informed pulses to be delivered. Similarly, IMD 110 may be configured to decrease the value of a control stimulation parameter that defines subsequent control pulses to be delivered.

IMD 110 may continue to decrease the informed stimulation parameter value and/or the control stimulation parameter value as long as the ECAP characteristic value continues to exceed the threshold ECAP characteristic value. Once, the informed and control stimulation parameters have been decreased, IMD 110 may attempt to increase the informed and control stimulation parameter values again back up to the predetermined first value intended for the informed stimulation pulses and/or control stimulation pulses. IMD 110 may be configured to determine a other characteristic values of subsequent ECAP signals elicited from control stimulation pulses delivered after sensing the first ECAP signal. In response to determining that another characteristic value of the subsequent ECAP signals decreases below the threshold ECAP characteristic value, IMD 110 may then increase the value of the informed and/or control stimulation parameter back up to a value limited to be less than or equal to the first value (e.g., back up to the predetermined value for the informed and/or control stimulation pulses that may be determined by a set of stimulation parameters or therapy program). In some examples, IMD 110 may iteratively increase the informed and/or control stimulation parameter values until the first value, or original value, is again reached after the characteristic values of the ECAP signal remain below the threshold ECAP characteristic value. IMD 110 may increase the informed and/or control stimulation parameter values at a slower rate than the informed and/or control stimulation parameter values are decreased, but, in other examples, IMD 110 may increase and decrease the informed and/or control stimulation parameters at the same rates.

As discussed herein, some example techniques for adjusting informed stimulation parameter values and/or control stimulation parameter values for electrical stimulation signals are based on comparing the value of a characteristic of a measured ECAP signal to a target ECAP characteristic value or using control parameter values at a determined target ECAP characteristic to inform adjustment of one or more control parameter values and/or informed parameter values to maintain the target ECAP according to known relationships between parameters. For example, during delivery of an electrical stimulation signal, IMD 110, via two or more electrodes interposed on leads 108, senses electrical potentials of tissue of the spinal cord 106 of patient 102 to measure the electrical activity of the tissue. IMD 110 senses ECAPs from the target tissue of patient 102, e.g., with electrodes on one or more leads 108 and associated sensing circuitry. In some examples, IMD 110 receives a signal indicative of the ECAP from one or more sensors, e.g., one or more electrodes and circuitry, internal or external to patient 102. Such an example signal may include a signal indicating an ECAP of the tissue of the patient 102. Examples of the one or more sensors include one or more sensors can measure a compound action potential of the patient 102, or a physiological effect indicative of a compound action potential. For example, to measure a physiological effect of a compound action potential, the one or more sensors may be an accelerometer, a pressure sensor, a bending sensor, a sensor can detect a posture of patient 102, or a sensor can detect a respiratory function of patient 102. However, in other examples, external programmer 104 receives a signal indicating a compound action potential in the target tissue of patient 102 and transmits a notification to IMD 110.

In the example of FIG. 1, IMD 110 described as performing a plurality of processing and computing functions. However, external programmer 104 instead may perform one, several, or all of these functions. In this alternative example, IMD 110 functions to relay sensed signals to external programmer 104 for analysis, and external programmer 104 transmits instructions to IMD 110 to adjust the one or more parameters defining the electrical stimulation signal based on analysis of the sensed signals. For example, IMD 110 may relay the sensed signal indicative of an ECAP to external programmer 104. External programmer 104 may compare the parameter value of the ECAP to the target ECAP characteristic value, and in response to the comparison, external programmer 104 may instruct IMD 110 to adjust one or more parameters that define the electrical stimulation signal.

In the example techniques described herein, the informed stimulation parameter values, control stimulation parameter values, growth curves, posture states, and the target ECAP characteristic values (e.g., values of the ECAP indicative of target stimulation intensity) may be initially set at the clinic but may be set and/or adjusted at home by patient 102. Once the target ECAP characteristic values are set, the example techniques allow for automatic adjustment of informed and/or control stimulation parameters to maintain consistent volume of neural activation and consistent perception of therapy for the patient when the electrode-to-neuron distance changes. The ability to change the stimulation parameter values may also allow the therapy to have long term efficacy, with the ability to keep the intensity of the stimulation (e.g., as indicated by the ECAP) consistent by comparing the measured ECAP values to the target ECAP characteristic value. IMD 110 may perform these changes without intervention by a physician or patient 102.

In some examples, the system may change the target ECAP characteristic value over a period of time (e.g., based on a sensed posture state or change in patient conditions). The system may be programmed to change the target ECAP characteristic in order to adjust the intensity of the informed pulses and/or control pulses to provide varying sensations to the patient (e.g., increase or decrease the volume of neural activation). In one example, a system may be programmed to oscillate a target ECAP characteristic value between a maximum target ECAP characteristic value and a minimum target ECAP characteristic value at a predetermined frequency to provide a sensation to the patient that may be perceived as a wave or other sensation that may provide therapeutic relief for the patient. The maximum target ECAP characteristic value, the minimum target ECAP characteristic value, and the predetermined frequency may be stored in the memory of IMD 110 and may be updated in response to a signal from external programmer 104 (e.g., a user request to change the values stored in the memory of IMD 110). In other examples, the target ECAP characteristic value may be programed to steadily increase or steadily decrease to a baseline target ECAP characteristic value over a period of time. In other examples, external programmer 104 may program the target ECAP characteristic value to automatically change over time according to other predetermined functions or patterns. In other words, the target ECAP characteristic value may be programmed to change incrementally by a predetermined amount or predetermined percentage, the predetermined amount or percentage being selected according to a predetermined function (e.g., sinusoid function, ramp function, exponential function, logarithmic function, or the like). Increments in which the target ECAP characteristic value is changed may be changed for every certain number of pulses or a certain unit of time. Although the system may change the target ECAP characteristic value, received ECAP signals may still be used by the system to adjust one or more informed and/or control parameter values of the electrical stimulation signal in order to meet the target ECAP characteristic value.

As described herein, IMD 110 can be configured to provide therapy to a patient (e.g., pain relief therapy) by using multimodal stimulation (e.g., differential targeted multiplexed stimulation). Multiple modal stimulation includes different pulse trains (e.g., a prime pulse train and a base pulse train) defined by different stimulation parameters such as different frequencies and different electrode combinations. Since different types of cells, such as glial cells and neurons, respond differently to electrical fields, it is then possible to differentially modulate the response of these cell populations with distinctly different electrical parameters. For example, the prime pulse train can be delivered to affect glial cells and the base pulse train can be delivered to affect neurons. Generally, the prime pulse train is delivered at a higher pulse frequency than the base pulse train, as described herein.

The oscillatory electromagnetic fields applied to neural structures can induce changes in synaptic plasticity upon modulation of two different cell populations: neurons and glial cells. This is concurrent with the effects on neurons such as action potential generation or blockade by the stimulation of mechanosensitive fibers to mask (or close the gate to) nociceptive signals travelling to the brain. In addition, glial cells are immunocompetent cells that constitute the most common cell population in the nervous system and play a fundamental role in the development and maintenance of chronic neuropathic pain. Glial cells are responsible for monitoring the status of the nervous system by using constant chemical communication with neurons and other glial cells. Microglia are the glial cells in charge of monitoring the brain and spinal cord. Following a nerve (or brain) injury, these cells become activated and respond to any stimulus that is considered a threat to Central Nervous System (CNS) homeostasis. This activation involves morphological changes in the microglia accompanied by changes in chemotaxis and phagocytic activity, as well as the release of chemokines and cytokines that induce a response from the immune system. It has been shown that microglia are the CNS immediate responders to injury. Injury also triggers the activation of astrocytes, glial cells that monitor the synaptic clefts and thus are involved in synaptic plasticity via the regulation of neuro and glial transmitter molecules and involvement of immune cells for synaptic pruning. Astrocyte activation and regulation is sustained for longer time and thus it can be hypothesized that astrocytes play an important role in changes affecting synaptic plasticity in chronic pain. There is experimental evidence that supports this hypothesis. It is worth noting that at the Peripheral Nervous System (PNS), oligodendrocytes, Schwann cells and satellite glial cells, similar to astroglia, play similar roles.

Calcium ions and phosphorylating processes mediated by ATP play an important role in glial response to injury. Electrical impulses induce changes in the concentration of calcium ions in the astrocytes, which propagates between astrocytes via calcium waves. This, in turn, signals the release of transmitters such as glutamate, adenosine and ATP, even after sodium channel blockade, which modulates both neuronal excitability and synaptic transmission. The presence of an external oscillatory electrical field then provides a stimulus for glial cells to affect synapses that have been negatively affected by injury. The electrical field provides a priming response that moves the function of the synapse towards a normal state.

Without being bound by theory, it is possible to electrically stimulate glial cells, for example, as their response (glial depolarization, release/uptake of ions, release of glial transmitters) depends on the specific parameters such as amplitude, frequency, phase polarity, waveform shape, and width (in the case of rectangular waveforms) of the stimulation. For example, the release of glutamate from astrocytes may be modulated in proportion to the amount of anodic current administered during biphasic pulsed stimulation. Monophasic cathodic stimulation of hippocampal astrocytes promotes the release of glutamate. The introduction of an anodic component decreases the amount of glutamate released. Given that the glial cells and neurons respond differently to electrical fields; it is then possible to differentially modulate the response of these cell populations with distinctly different electrical parameters. This theory sets a mechanistic basis of multimodal stimulation. Subthreshold stimulation with an electromagnetic field set at an optimum frequency, amplitude, waveform, width and phase may modulate the behavior of glial cells and the way they interact with neurons at the synaptic level. Thus, multimodal modulation provides the ability to control the balance of glutamate and glutamine in a calcium dependent manner and the possibility of modulating such balance in the appropriate manner with electromagnetic fields.

Electromagnetic fields modulate the expression of genes and proteins, which are involved in many processes involving synaptic plasticity, neuroprotection, neurogenesis, and inflammation. A genome-wide expression analysis of ipsilateral DC and DRG tissues obtained from an animal model of chronic neuropathic pain, in which SCS was applied continuously for 72 hours, provided findings that informed development of the multimodal methodologies described below. Without wishing to be bound by theory, the gene expression results indicated that the analgesic effect was likely induced at the molecular level in addition to, or independently of, the electric field blocking or masking nerve signaling. For example, SCS was identified to have upregulated genes for calcium binding proteins (Cabp), cytokines (Tnf, 116, 111b, Cxcl16, 1fg), cell adhesion (1tgb) and specific immune response proteins (Cd68, Tlr2), all of which have been linked to glial activation. Modulation parameters, particularly the oscillation frequency and amplitude, may play an important role in the mode of action.

In some examples, a system may deliver multimodal modulation which utilizes a composite electric field with at least one component oscillating at a frequency higher than the other component. This composite electric field is believed to provide pain relief that exceeds the amount of pain relief provided by either electric field on its own. The electrical field of the higher frequency "priming" component provides a persistent electrochemical potential that may facilitate the stimulation of nerves by another component that is oscillating at a lower frequency. Without being bound by theory, the priming component can lower the threshold for depolarization of nerve fibers while simultaneously modulating glial activation. The priming component may also lower the impedance of the stimulated tissue, which can enable for better penetration of the electric field into the neural tissue. The frequent pulsing of the priming component also contributes to a lower threshold for depolarization of nerve fibers via membrane integration of the electrical stimulus. Additionally, the priming component may contribute to neuronal desynchronization, which is a mechanism that helps with the reestablishment of neuronal circuits that have been unnaturally synchronized to maintain a nociceptive input into the brain.

In the prime multimodal modulation technique, a mechanism of depolarization is combined with amplitudes lower or slightly higher than the Paresthesia Threshold (PT) or perception threshold, so the patient may or may not experience tingling even though tonic stimulation is being applied. In certain embodiments, the composite signal, including the primary component that provides electrical stimulation at higher than the tonic frequencies, may activate the molecular mechanisms that allow for resetting of the synaptic plasticity to a state closer to the one previous to central sensitization induced by injury, thus providing a mechanism for long lasting pain relief.

In certain embodiments, the Priming Frequency (PF) may be set to any frequency between 100 Hz to 1200 kHz. However, higher or lower frequencies may be used in other examples. When a charged-balanced pulsed rectangular electrical component, e.g., biphasic symmetric, biphasic asymmetric, capacitor coupled monophasic, is used, the Pulse Width (PW) of the priming component may be set as low as 10 us and as large as allowed by the priming frequency. In some examples, the PW of pulses may be between approximately 150 to 300 $\mu$s, although other examples may have smaller or larger pulse widths. Either a voltage or current controlled composite signal may be used, although a current controlled signal may be more desirable as such signal does not depend on temporal impedance variations in the tissue being stimulated.

In certain embodiments, a first or priming frequency is between 50 Hz and 1200 Hz (burst), or between 150 Hz and 900 Hz (average). According to embodiments, multiple signals can be multiplexed within a repeating set of N pulse spaces. Each pulse space within the pattern can correspond to a different electrical signal with respective parameters. The lower average frequency can be generated by multiplexing a second, tonic signal component in one of the N pulses. According to embodiments, the burst frequency of the priming frequency signal component can be an integer multiple (M) of the tonic signal frequency such that the tonic pulse space only includes a pulse every M times the N set of pulse spaces are repeated. The blank pulse space results in a burst of N-1 pulses at the "burst" frequency, followed by a "missed" pulse resulting in a lower "average" frequency over the set of N pulses. As used herein, the average frequency of the priming signal is calculated separate without including pulses associated with the tonic signal. In some embodiments, the priming signal can be delivered to a different physical location using a different set of electrodes relative to the tonic signal. In another exemplary embodiment, the first or priming frequency is set to 1200 Hz (burst), or 900 Hz (average). In certain embodiments, each pulse within a burst may be provided on a separate program for different groups of electrodes, with a configuration set to allow for individual amplitude variability. Therefore, different pulse trains (e.g., a priming pulse train and a tonic pulse train) may be interleaved and delivered to provide the multimodal stimulation therapy.

In further exemplary embodiments, a second or tonic component is set at a frequency of about 50 Hz, interleaved into the treatment to account for the average priming frequency, though other tonic values and ranges are contemplated herein, e.g., 20 Hz to 2.00 Hz, 20 Hz to 100 Hz, 30 Hz to 80 Hz, etc. As discussed herein, the IMD 110 may be configured to sensed ECAP signals after one or more pulses of the tonic component (e.g., a base pulse train) and before the next subsequent pulse of the tonic component to inform any adjustments to a parameter defining the priming stimulation or other aspect of the overall multimodal stimulation or closed-loop control of the stimulation.

Disclosed herein are apparatus and methods for managing pain in a patient by using multimodal stimulation of neural structures, with an electromagnetic signal having multiple components of characteristic frequencies, amplitudes, and phase polarities. Moreover, IMD 110 may be configured to provide automatic closed-loop control of such stimulation based on sensed ECAP signals. Multimodal modulation for pain management, in accordance with the disclosure, contemplates the use of oscillating electromagnetic fields which is applied via an array of electrodes (referred as contacts or leads) to a particular neural structure using temporal and amplitude characteristics, to modulate glial and neuronal interactions as the mechanism for relieving chronic pain. More specifically, exemplary aspects provide an apparatus and method for modulating the expression of genes involved in diverse pathways including inflammatory/immune system mediators, ion channels and neurotransmitters, in both the Spinal Cord (SC) and Dorsal Root Ganglion (DRG). In one exemplary embodiment, such expression modulation is caused by spinal cord stimulation or peripheral nerve stimulation. In one embodiment, the amplitudes and frequencies of the signal or signals used to create the multimodal stimulation of neural structures may be optimized for pain relief and low power usage in an implantable multimodal signal generator, as described herein.

According to one exemplary embodiment, apparatuses and methods provide for managing pain in a patient by using multiplexed stimulation signals to target different neural structures such that the multiple stimulation signals are multiplexed in the time domain, hereafter referred to as multimodal stimulation or "differential target multiplexed stimulation." For instance, a signal generator can multiplex signals (e.g., pulses from different pulse trains) that can have different signal characteristics (e.g., pulse frequency, amplitude, or pulse duration) to generate differential target multiplexed stimulation for pain management. In accordance with aspects of the disclosure, the output of the signal generator can be used to produce separate oscillating electromagnetic fields (stimulation signals, such as pulses or continuous signals) which can be applied to different set of a plurality of electrodes (also referred as contacts). The electrodes can be part of a lead that is designed to apply the respective stimulation signals to different parts of a particular neural structure.

Various aspects of the disclosure relate to the use of a variety of temporal and amplitude characteristics in order to modulate glial and neuronal interactions as the mechanism for relieving chronic pain. The multiplexed stimulation signals have characteristics that allow for a synergistic targeting of glial cells and neurons in a differential manner. For instance, IMD 200 can modulate the expression of genes and proteins involved in diverse pathways, including inflammatory/immune system mediators, ion channels and neurotransmitters, associated with the interaction of glia and neurons in neural tissue. In embodiments, such expression modulation may be caused by any of spinal cord stimulation, dorsal root ganglion stimulation, brain stimulation, or peripheral nerve stimulation. In some embodiments, the amplitudes, phase polarity, waveforms, and frequencies of the signals combined to create the differential target multiplexed stimulation of neural structures may be optimized for pain relief and low power usage in an implantable signal generator, as described herein.

In an example of differential target multiplexed stimulation therapy, a set of high frequency charge-balanced biphasic pulsed signals (e.g., one or more first pulse trains or prime pulse trains) in which the polarity of the first phase of the high frequency signals may be either cathodic or anodic is utilized. In examples, a set of low frequency signals (e.g., one or more second pulse trains or base pulse trains) is used that may have waveform characteristics different from those of the high frequency signals. The polarity of the first phase of the biphasic charge-balanced low frequency signals may be either cathodic or anodic. The high and low frequency stimulation signals can be delivered to the neural tissues by multiplexing individual pulses from each via respective sets of electrodes. In certain embodiments, the respective sets of electrodes can be co-located in close proximity to the same neural tissue (e.g., near the same vertebrae).

Although electrical stimulation is generally described herein in the form of electrical stimulation pulses, electrical stimulation may be delivered in non-pulse form in other examples. For example, electrical stimulation may be delivered as a signal having various waveform shapes, frequencies, and amplitudes. Therefore, electrical stimulation in the form of a non-pulse signal may be a continuous signal than may have a sinusoidal waveform or other continuous waveform.

In the case of multimodal modulation of the spinal cord, various multi-contact leads can be positioned in the epidural space to stimulate the cell populations already described. In one particular arrangement, the leads can be positioned parallel to each other, although not necessarily coplanar within the epidural space. Two eight-contact electrode arrays can be used for the disclosed multimodal modulation techniques. Note that the polarity of the leads can also be customized during the programming stage, either as bipolar, monopolar, or guarded cathode configurations. Another example of a possible electrode array arrangement includes leads arranged staggered relative to each other. The customization and optimization of therapy may comprise the positioning of the leads within the epidural space at appropriate vertebral segments in either type of lead arrangement.

Other arrangements may be used to stimulate different places along the spinal canal, e.g., the leads do not need to be parallel. For example, in one arrangement, one lead can be dedicated to deliver a signal at the spinal cord at a given vertebral level, while the other provides a signal either more caudad or cephalad relative to the position of the other lead. Leads can be, in principle, located at any vertebral level in the spinal cord, or could also be positioned peripherally, because the principle behind multimodal modulation applies to peripheral glial cells that survey the axons.

Furthermore, the multimodal stimulation electromagnetic fields location and penetration may be also utilized for customization and optimization of therapy by delivering multimodal stimulation signals to particular arrays of electrodes within each lead by setting monopolar, bipolar, or guarded cathode arrangements of such electrode arrays. For example, therapy for a patient with low back pain that extends into one of the lower extremities may require positioning the stimulation leads in a staggered arrangement within the epidural space along vertebral levels thoracic 8 (TS) and thoracic 12 (T12). An array of electrodes in the more cephalad of the leads may be set to monopolar, bipolar or guarded cathode arrangement. Another array of electrodes in the more caudad of the leads may be set to monopolar, bipolar or guarded cathode arrangement. The clinician will be able to customize the electrode array setting in a methodical manner such that therapy can be optimized for based on feedback from the patient.

Optionally, pain relief may also be used by position the leads in the neighborhood of a peripheral nerve. Peripheral Nerve Stimulation (PNS) is an alternative therapy for chronic pain in which a target nerve has been identified to be the source of pain. The current understanding of the therapeutic effects of PNS is also based on the gate control theory. However, axons of sensory neurons in peripheral nerves are surrounded by glial cells that are known to respond accordingly to the frequency characteristics of a stimulus.

Multimodal peripheral nerve stimulation involves the positioning of one or more stimulation leads around or in the neighborhood of a target nerve. The leads are connected to a signal generator with multimodal capacity as described herein. Multimodal stimulation is delivered to the neural tissue consisting of neuron axons and their corresponding glial cells (Schwann cells) according to the principles and methods described in this application. The leads may be implanted and positioned around the target nerve using an invasive surgical approach or percutaneously utilizing a needle cannula.

Alternatively, as would be the case for the stimulation of target nerves that are close to the skin surface (such as the vagus nerve, nerves in the joints of the extremities, etc.) the leads may be arranged inside a conductive biocompatible pad for delivery of the multimodal electromagnetic field transcutaneously. This embodiment constitutes Transcutaneous Electrical Nerve Multimodal Stimulation (TENMS). In this embodiment, the priming high frequency component of the multimodal signal lowers the impedance of the skin and subcutaneous tissue and allows for better penetration of the tonic signal. The priming signal also provides a modulating signal for perisytiaptic glial cells in the neuromuscular junction. These cells are known to discriminate different stimulation patterns and respond accordingly, thus allowing for modulation of the synapse with multimodal stimulation. The tonic component of the multimodal signal is used to stimulate the neuronal axon at lower thresholds.

Although in one example IMD 1 10 takes the form of an SCS device, in other examples, IMD 110 takes the form of any combination of deep brain stimulation (DBS) devices, implantable cardioverter defibrillators (ICDs), pacemakers, cardiac resynchronization therapy devices (CRT-Ds), left ventricular assist devices (LVADs), implantable sensors, orthopedic devices, or drug pumps, as examples.

FIG. 2 is a block diagram of IMD 2.00. IMD 2.00 may be an example of IMD 110 of FIG. 1. In the example shown in FIG. 2, IMD 200 includes switch circuitry 202, stimulation generation circuitry 204, sensing circuitry 206, processing circuitry 208, sensor 210, telemetry circuitry 212, power source 214, and memory 216. Each of these circuits may be or include programmable or fixed function circuitry can perform the functions attributed to respective circuitry. For example, processing circuitry 208 may include fixed-function or programmable circuitry, stimulation generation circuitry 204 may include circuitry can generate electrical stimulation signals such as pulses or continuous waveforms on one or more channels, sensing circuitry 206 may include sensing circuitry for sensing signals, and telemetry circuitry 212 may include telemetry circuitry for transmission and reception of signals. Memory 216 may store computer-readable instructions that, when executed by processing circuitry 208, cause IMD 200 to perform various functions described herein. Memory 216 may be a storage device or other non-transitory medium.

In the example shown in FIG. 2, memory 216 stores patient data 218, which may include anything related to the patient such as one or more patient postures, an activity level, or a combination of patient posture and activity level. Memory 216 may store stimulation parameter settings 220 within memory 216 or separate areas within memory 216. Each stored stimulation parameter setting 220 defines values for one or more sets of electrical stimulation parameters (e.g., an informed stimulation parameter set and a control stimulation parameter set, or parameters for other pulse trains), such as pulse amplitude, pulse width, pulse frequency, electrode combination, pulse burst rate, pulse burst duration, and/or waveform shape. Stimulation parameter settings 220 may also include additional information such as instructions regarding delivery of electrical stimulation signals based on stimulation parameter relationship data, which can include relationships between two or more stimulation parameters based upon data from electrical stimulation signals delivered to patient 102 or data transmitted from external programmer 104. The stimulation parameter relationship data may include measurable aspects associated with stimulation, such as an ECAP characteristic value. Stimulation parameter settings 220, or another portion of memory 216, may include instructions on how processing circuitry 208 can modulate informed stimulation parameters and/or control stimulation parameters based on the detected posture state and/or at least one of a target ECAP characteristic value or a threshold ECAP characteristic value, as described herein.

Memory 216 also stores patient ECAP characteristics 222 which may include target ECAP characteristics and/or threshold ECAP characteristic values determined for the patient and/or a history of measured ECAP characteristic values for the patient. Memory 216 may also control policy data 224 in separate areas from or as part of patient stimulation parameter settings. Control policy data 224 may include instructions that processing circuitry 208 uses for how to adjust one or more parameter values that define stimulation in response to ECAP characteristic values. Memory 216 may include gain values that processing circuitry 208 may use to modulate informed and/or control stimulation pulses as described herein.

Accordingly, in some examples, stimulation generation circuitry 204 generates electrical stimulation signals (e.g., informed pulses and control pulses) in accordance with the electrical stimulation parameters noted above. Other ranges of stimulation parameter values may also be useful and may depend on the target stimulation site within patient 102. While stimulation pulses are described, stimulation signals may be of any form, such as continuous-time signals (e.g., sine waves) or the like. Switch circuitry 202 may include one or more switch arrays, one or more multiplexers, one or more switches (e.g., a switch matrix or other collection of switches), or other electrical circuitry configured to direct stimulation signals from stimulation generation circuitry 204 to one or more of electrodes 232, 234, or directed sensed signals from one or more of electrodes 232, 234 to sensing circuitry 206. In other examples, stimulation generation circuitry 204 and/or sensing circuitry 206 may include sensing circuitry to direct signals to and/or from one or more of electrodes 232, 234, which may or may not also include switch circuitry 202.

Sensing circuitry 206 may be configured to monitor signals from any combination of electrodes 232, 234. In some examples, sensing circuitry 206 includes one or more amplifiers, filters, and analog-to-digital converters. Sensing circuitry 206 may be used to sense physiological signals, such as ECAPs. In some examples, sensing circuitry 206 detects ECAPs from a particular combination of electrodes 232, 234. In some cases, the particular combination of electrodes for sensing ECAPs includes different electrodes than a set of electrodes 232, 234 used to deliver control stimulation pulses and/or informed stimulation pulses. Alternatively, in other cases, the particular combination of electrodes used for sensing ECAPs includes at least one of the same electrodes as a set of electrodes used to deliver informed and/or control stimulation pulses to patient 102. Sensing circuitry 206 may provide signals to an analog-to-digital converter, for conversion into a digital signal for processing, analysis, storage, or output by processing circuitry 208.

Processing circuitry 208 may include any one or more of a microprocessor, a controller, a digital signal processor (DSP), an application specific integrated circuit (ASIC), a field-programmable gate array (FPGA), discrete logic circuitry, or any other processing circuitry can provide the functions attributed to processing circuitry 208 herein may be embodied as firmware, hardware, software or any combination thereof. Processing circuitry 208 controls stimulation generation circuitry 204 to generate electrical stimulation signals according to stimulation parameter settings 220 stored in memory 216 to apply stimulation parameter values, such as pulse amplitude, pulse width, pulse frequency, and waveform shape of each of the electrical stimulation signals.

In the example shown in FIG. 2, the set of electrodes 232 includes electrodes 232A, 232B, 232C, and 232D, and the set of electrodes 234 includes electrodes 234A, 234B, 234C, and 234D. In other examples, a single lead may include all eight electrodes 232 and 234 along a single axial length of the lead. Processing circuitry 208 also controls stimulation generation circuitry 204 to generate and apply the electrical stimulation signals to selected combinations of electrodes 232, 234. In some examples, stimulation generation circuitry 104 includes a switch circuit (instead of, or in addition to, switch circuitry 202) that may couple stimulation signals to selected conductors within leads 230, which, in turn, deliver the stimulation signals across selected electrodes 232, 234. Such a switch circuit may be a switch array, switch matrix, multiplexer, or any other type of switch circuitry can selectively couple stimulation energy to selected electrodes 232, 234 and to selectively sense bioelectrical neural signals of a spinal cord of the patient (not shown in FIG. 2) with selected electrodes 232, 234.

In other examples, however, stimulation generation circuitry 204 does not include a switch circuit and switch circuitry 202 does not interface between stimulation generation circuitry 204 and electrodes 232, 234. In these examples, stimulation generation circuitry 204 comprises a plurality of pairs of voltage sources, current sources, voltage sinks, or current sinks connected to each of electrodes 232, 234 such that each pair of electrodes has a unique signal circuit. In other words, in these examples, each of electrodes 232, 234 is independently controlled via its own signal circuit (e.g., via a combination of a regulated voltage source and sink or regulated current source and sink), as opposed to switching signals between electrodes 232, 234.

Electrodes 232, 234 on respective leads 230 may be constructed of a variety of different designs. For example, one or both of leads 230 may include one or more electrodes at each longitudinal location along the length of the lead, such as one electrode at different perimeter locations around the perimeter of the lead at each of the locations A, B, C, and D. In one example, the electrodes may be electrically coupled to stimulation generation circuitry 204, e.g., via switch circuitry 202 and/or switch circuitry of the stimulation generation circuitry 204, via respective wires that are straight or coiled within the housing of the lead and run to a connector at the proximal end of the lead. In another example, each of the electrodes of the lead may be electrodes deposited on a thin film. The thin film may include an electrically conductive trace for each electrode that runs the length of the thin film to a proximal end connector. The thin film may then be wrapped (e.g., a helical wrap) around an internal member to form the lead 230. These and other constructions may be used to create a lead with a complex electrode geometry.

Although sensing circuitry 206 is incorporated into a common housing with stimulation generation circuitry 204 and processing circuitry 208 in FIG. 2, in other examples, sensing circuitry 206 may be in a separate housing from IMD 200 and may communicate with processing circuitry 208 via wired or wireless communication techniques.

In some examples, one or more of electrodes 232 and 234 may be suitable for sensing ECAPs. For instance, electrodes 232 and 234 may sense the voltage amplitude of a portion of the ECAP signals, where the sensed voltage amplitude is a characteristic the ECAP signal.

Memory 216 may be configured to store information within IMD 200 during operation. Memory 216 may include a computer-readable storage medium or computer-readable storage device. In some examples, memory 216 includes one or more of a short-term memory or a long-term memory. Memory 216 may include, for example, random access memories (RAM), dynamic random access memories (DRAM), static random access memories (SRAM), magnetic discs, optical discs, flash memories, or forms of electrically programmable memories (EPROM) or electrically erasable and programmable memories (EEPROM). In some examples, memory 216 is used to store data indicative of instructions for execution by processing circuitry 208. As discussed herein, memory 216 can store patient data 218, stimulation parameter settings 220, patient ECAP characteristics 222, and control policy data 224.

Sensor 210 may include one or more sensing elements that sense values of a respective patient parameter. As described, electrodes 232 and 234 may be the electrodes that sense, via sensing circuitry 206, a value of the ECAP indicative of a target stimulation intensity at least partially caused by a set of control stimulation parameter values. Sensor 210 may include one or more accelerometers, optical sensors, chemical sensors, temperature sensors, pressure sensors, or any other types of sensors. Sensor 210 may output patient parameter values that may be used as feedback to control delivery of electrical stimulation signals. IMD 200 may include additional sensors within the housing of IMD 200 and/or coupled via one of leads 108 or other leads. In addition, IMD 200 may receive sensor signals wirelessly from remote sensors via telemetry circuitry 212, for example. In some examples, one or more of these remote sensors may be external to patient (e.g., carried on the external surface of the skin, attached to clothing, or otherwise positioned external to the patient). In some examples, signals from sensor 210 may indicate a posture state (e.g., sleeping, awake, sitting, standing, or the like), and processing circuitry 208 may select target and/or threshold ECAP characteristic values according to the indicated posture state.

Telemetry circuitry 212 supports wireless communication between IMD 200 and an external programmer (not shown in FIG. 2) or another computing device under the control of processing circuitry 208. Processing circuitry 208 of IMD 200 may receive, as updates to programs, values for various stimulation parameters such as amplitude and electrode combination (e.g., for informed and/or control pulses), from the external programmer via telemetry circuitry 212. Updates to stimulation parameter settings 220 and input efficacy threshold settings 226 may be stored within memory 216. Telemetry circuitry 212 in IMD 200, as well as telemetry circuits in other devices and systems described herein, such as the external programmer, may accomplish communication by radiofrequency (RF) communication techniques. In addition, telemetry circuitry 212 may communicate with an external medical device programmer (not shown in FIG. 2) via proximal inductive interaction of IMD 200 with the external programmer. The external programmer may be one example of external programmer 104 of FIG. 1. Accordingly, telemetry circuitry 212 may send information to the external programmer on a continuous basis, at periodic intervals, or upon request from IMD 110 or the external programmer.

Power source 214 delivers operating power to various components of IMD 200. Power source 214 may include a rechargeable or non-rechargeable battery and a power generation circuit to produce the operating power. Recharging may be accomplished through proximal inductive interaction between an external charger and an inductive charging coil within IMD 200. In other examples, traditional primary cell batteries may be used. In some examples, processing circuitry 208 may monitor the remaining charge (e.g., voltage) of power source 214 and select stimulation parameter values that may deliver similarly effective therapy at lower power consumption levels when needed to extend the operating time of power source 214. For example, power source 214 may switch to a lower pulse frequency based on the relationships of parameters that may provide similar ECAP characteristic values.

According to the techniques of the disclosure, stimulation generation circuitry 204 of IMD 200 receives, via telemetry circuitry 212, instructions to deliver electrical stimulation according to stimulation parameter settings 220 to a target tissue site of the spinal cord of the patient via a plurality of electrode combinations of electrodes 232, 234 of leads 230 and/or a housing of IiVID 200. Each electrical stimulation signal may elicit an ECAP that is sensed by sensing circuitry 206 via electrodes 232 and 234. Processing circuitry 208 may receive, via an electrical signal sensed by sensing circuitry 206, information indicative of an ECAP signal (e.g., a numerical value indicating a characteristic of the ECAP in electrical units such as voltage or power) produced in response to the electrical stimulation signal(s). Stimulation parameter settings 220 may be updated according to the ECAPs recorded at sensing circuitry 206 according to the following techniques.

In some examples, the pulse width of informed pulses are greater than the pulse width of control pulses. This difference in pulse width may allow ECAPs elicited from the control pulses to be detectable by the system when the longer pulse widths of the informed pulses prevent elicited ECAPs, or at least some portion of the elicited ECAPs, from being detectable. In some examples, the plurality of informed stimulation pulses are defined by an informed pulse width greater than approximately 300 microseconds and less than approximately 1000 microseconds, while the plurality of control stimulation pulses are defined by a control pulse width less than approximately 300 microseconds. In one example, the plurality of informed pulses each have a pulse width of greater than approximately 300 $\mu$s and less than approximately 2000 $\mu$s (i.e., 2 milliseconds). In some examples, the informed pulse width is greater than approximately 300 $\mu$s and less than approximately 900 $\mu$s. In another example, the informed pulse width is greater than approximately 300 $\mu$s and less than approximately 500 $\mu$s. In one example, the informed pulses have a pulse width of approximately 450 $\mu$s and

a pulse frequency of approximately 60 Hertz. Amplitude (current and/or voltage) for the pulses may be between approximately 0.5 mA (or volts) and approximately 10 mA (or volts), although amplitude may be lower or greater in other examples.

In some examples, the pulse frequency of the informed pulses (e.g., the prime pulse train) may prevent IMD 200 from sensing ECAP signals elicited by an informed pulse because the next informed pulse is delivered before the ECAP signal can be sensed. In this manner, the predetermined pulse frequency of the plurality of informed pulses may be less than approximately 1200 Hz and greater than 100 Hz. In other examples, the predetermined pulse frequency of the plurality of pulses of the informed pulses may be less than 100 Hz, such as between approximately 50 Hertz and 70 Hertz. In addition, the informed pulses may be delivered in bursts of pulses, with interburst frequencies of the informed pulses being low enough such that a sensed ECAP elicited by a control pulse can still fit within the window between consecutive pulses delivered within the burst of pulses. In any example, processing circuity 208 may be configured to detect ECAPs elicited from respective control stimulation pulses.

In some examples, the pulse width of the control pulses may be shorter than the pulse width of the informed pulses to reduce or prevent a sensed electrical artifact from control pulses from obscuring the ECAP signals (put another way, the pulse width of the informed pulses may be longer than the pulse width of the control pulses). For example, the control pulses may be less than approximately 300 microseconds ($\mu$s). In one example, the control pulse may be a bi-phasic pulse having a positive phase of approximately 100 $\mu$s and a negative phase of approximately 100 $\mu$s separated by an interphase interval of approximately 30 $\mu$s. In this manner, stimulation electrodes at one end of a lead may deliver the control pulse and electrodes at the other end of the same lead may sense the ECAP signal without, or with minimal, interference from the control pulse itself. In general, the term "pulse width" herein refers to the collective duration of every phase, and interphase interval when appropriate, of a single pulse. A single pulse may include a single phase in some examples (i.e., a monophasic pulse) or two or more phases in other examples (e.g., a bi-phasic pulse or a tri-phasic pulse). The pulse width defines a period of time beginning with a start time of a first phase of the pulse and concluding with an end time of a last phase of the pulse (e.g., a biphasic pulse having a positive phase lasting 100 $\mu$s, a negative phase lasting 100 $\mu$s, and an interphase interval lasting 30 $\mu$s defines a pulse width of 230 $\mu$s). In some examples, the pulse frequency of the control pulses (e.g., the base pulse train) may be less than approximately 100 Hz. In one example, the pulse frequency of the control pulses may be selected in a range from approximately 40 Hz to 60 Hz. In this manner, ECAP signals may be sensed between consecutive pulses.

Processing circuitry 208 may be configured to compare one or more characteristics of ECAPs sensed by sensing circuitry 206 with target ECAP characteristics stored in memory 216 (e.g., patient ECAP characteristics 222). For example, processing circuitry 208 can determine the amplitude of each ECAP signal received at sensing circuitry 206, and processing circuitry 208 can determine the representative amplitude of at least one respective ECAP signal and compare the representative amplitude of a series of ECAP signals to a target ECAP characteristic value.

In other examples, processing circuitry 208 may use the representative amplitude of the at least one respective ECAP to change other parameters of stimulation pulses (e.g., informed pulses and/or control pulses) to be delivered, such as pulse width, pulse frequency, and pulse shape. All of these parameters may contribute to the intensity of the stimulation pulses, and changing one or more of these parameter values may effectively adjust the stimulation pulse intensity to compensate for the changed distance between the stimulation electrodes and the nerves indicated by the characteristic (e.g., a representative amplitude) of the ECAP signals.

In some examples, leads 230 may be linear 8-electrode leads (not pictured); sensing and stimulation delivery may each be performed using a different set of electrodes. In a linear 8-electrode lead, each electrode may be numbered consecutively from 0 through 7. For instance, a pulse may be generated using electrode 1 as a cathode and electrodes 0 and 2 as anodes (e.g., a guarded cathode), and a respective ECAP signal may be sensed using electrodes 6 and 7, which are located on the opposite end of the electrode array. This strategy may minimize the interference of the stimulation pulse with the sensing of the respective ECAP. Other electrode combinations may be implemented, and the electrode combinations may be changed using the patient programmer via telemetry circuitry 212. For example, stimulation electrodes and sensing electrodes may be positioned closer together. Shorter pulse widths for the nontherapeutic pulses may allow the sensing electrodes to be closer to the stimulation electrodes.

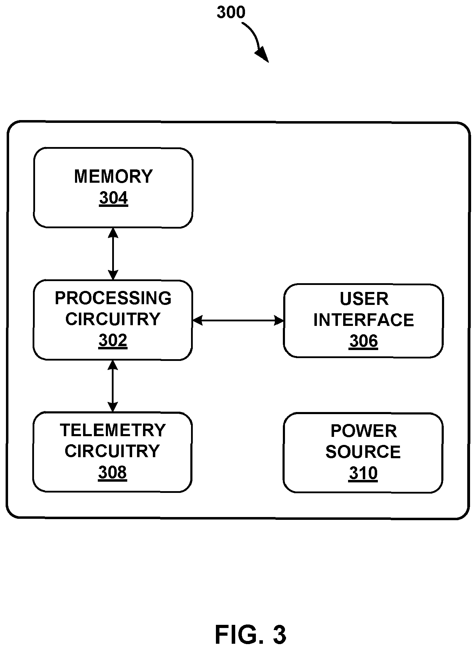

FIG. 3 is a block diagram of the example external programmer 300. External programmer 300 may be an example of external programmer 104 of FIG. 1. Although programmer 300 may generally be described as a hand-held device, external programmer 300 may be a larger portable device or a more stationary device. In addition, in some examples, external programmer 300 may be included as part of an external charging device or include the functionality of an external charging device. As illustrated in FIG. 3, external programmer 300 may include a processing circuitry 302, memory 304, user interface 306, telemetry circuitry 308, and power source 310. Storage device 304 may store instructions that, when executed by processing circuitry 302, cause processing circuitry 302 and external programmer 300 to provide the functionality ascribed to external programmer 300 throughout this disclosure. Each of these components, circuitry, or modules, may include electrical circuitry that can perform some, or all of the functionality described herein. For example, processing circuitry 302 may include processing circuitry to perform the processes discussed with respect to processing circuitry 302.

In general, programmer 300 comprises any suitable arrangement of hardware, alone or in combination with software and/or firmware, to perform the techniques attributed to programmer 300, and processing circuitry 302, user interface 306, and telemetry circuitry 308 of programmer 300. In various examples, programmer 300 may include one or more processors, such as one or more microprocessors, DSPs, ASICs, FPGAs, or any other equivalent integrated or discrete logic circuitry, as well as any combinations of such components. Programmer 300 also, in various examples, may include a memory 304, such as RAM, ROM, PROM, EPROM, EEPROM, flash memory, a hard disk, a CD-ROM, comprising executable instructions for causing the one or more processors to perform the actions attributed to them. Moreover, although processing circuitry 302 and telemetry circuitry 308 are described as separate, in some examples, processing circuitry 302 and telemetry circuitry 308 are functionally integrated. In some examples, processing circuitry 302 and telemetry circuitry 308 correspond to individual hardware units, such as ASICs, DSPs, FPGAs, or other hardware units.

Memory 304 (e.g., a storage device) may store instructions that, when executed by processing circuitry 302, cause processing circuitry 302 and programmer 300 to provide the functionality ascribed to programmer 300 throughout this disclosure. For example, memory 304 may include instructions that cause processing circuitry 302 to obtain a stimulation parameter setting from memory, select a spatial electrode movement pattern, or receive a user input and send a corresponding command to programmer 300, or instructions for any other functionality. In addition, memory 304 may include a plurality of stimulation parameter settings, where each setting includes a parameter set that defines electrical stimulation. Memory 304 may also store data received from a medical device (e.g., IMD 110). For example, memory 304 may store ECAP related data recorded at a sensing circuitry of the medical device, and memory 304 may also store data from one or more sensors of the medical device.

User interface 306 may include a button or keypad, lights, a speaker for voice commands, a display, such as a liquid crystal (LCD), light-emitting diode (LED), or organic light-emitting diode (OLED). In some examples the display may be a touch screen. User interface 306 can display any information related to the delivery of electrical stimulation, identified patient behaviors, sensed patient parameter values, patient behavior criteria, or any other such information. External programmer 300 may receive user input (e.g., indication of when the patient changes posture states) via user interface 306. The input may be, for example, in the form of pressing a button on a keypad or selecting an icon from a touch screen. The input may request starting or stopping electrical stimulation, the input may request a new spatial electrode movement pattern or a change to an existing spatial electrode movement pattern, of the input may request some other change to the delivery of electrical stimulation. In some examples, user interface 306 may receive user input requesting to adjust a stimulation parameter value. In other examples, user interface 306 may receive input from the patient and/or clinician regarding efficacy of the therapy, such as binary feedback, numerical ratings, textual input, etc. In some examples, processing circuitry 302 may interpret patient requests to change therapy as negative feedback regarding the current parameter values used to define therapy.

Telemetry circuitry 308 may support wireless communication between the medical device and programmer 300 under the control of processing circuitry 302. Telemetry circuitry 308 can communicate with another computing device via wireless communication techniques, or direct communication through a wired connection. In some examples, telemetry circuitry 308 provides wireless communication via an RF or proximal inductive medium. In some examples, telemetry circuitry 308 includes an antenna, which may take on a variety of forms, such as an internal or external antenna.

Examples of local wireless communication techniques that may be employed to facilitate communication between programmer 300 and IMD 110 include RF communication according to the 902.11 or Bluetooth specification sets or other standard or proprietary telemetry protocols. In this manner, other external devices may be capable of communicating with programmer 300 without needing to establish a secure wireless connection. As described herein, telemetry circuitry 308 can transmit a spatial electrode movement pattern or other stimulation parameter values to IMD 110 for delivery of electrical stimulation.

In some examples, selection of stimulation parameter settings (e.g., informed parameter values and/or control parameter values) may be transmitted to the medical device for delivery to the patient. In other examples, stimulation parameter settings may include medication, activities, or other instructions that the patient must perform themselves or a caregiver perform for patient 102. In some examples, external programmer 300 may provide visual, audible, and/or tactile notifications that indicate there are new instructions. External programmer 300 may require receiving user input acknowledging that the instructions have been completed in some examples.

Power source 310 can deliver operating power to various components of programmer 300. Power source 310 may be the same as or substantially similar to power source 214. Power source 310 may include a battery and a power generation circuit to produce the operating power. In some examples, the battery is rechargeable to allow extended operation. Recharging may be accomplished by electrically coupling power source 310 to a cradle or plug that is connected to an alternating current (AC) outlet. In addition, recharging may be accomplished through proximal inductive interaction between an external charger and an inductive charging coil within external programmer 300. In other examples, traditional batteries (e.g., nickel cadmium or lithium ion batteries) may be used. In addition, external programmer 300 may be directly coupled to an alternating current outlet to operate.

The architecture of external programmer 300 illustrated in FIG. 3 is shown as an example. The techniques as set forth in this disclosure may be implemented in the example external programmer 300 of FIG. 3, as well as other types of systems not described specifically herein. Nothing in this disclosure should be construed so as to limit the techniques of this disclosure to the example architecture illustrated by FIG. 3.

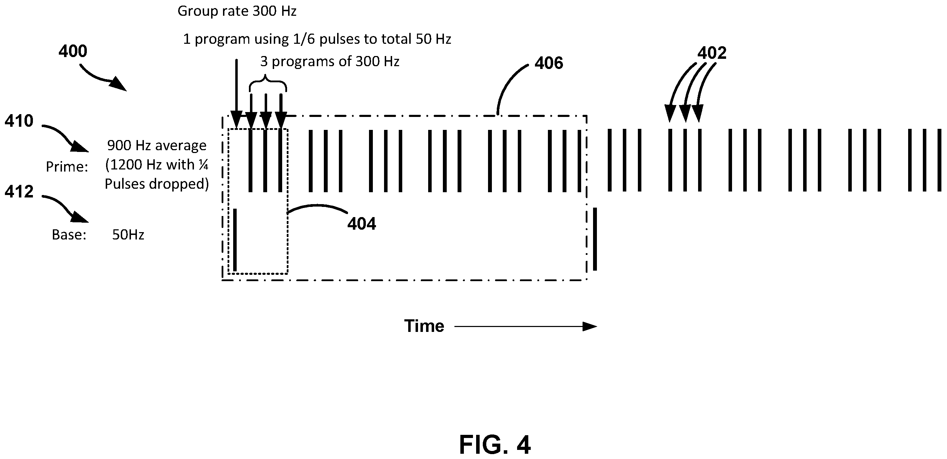

FIG. 4 is a timing diagram illustrating an example of electrical stimulation pulses (e.g., different pulse trains) delivered according to different stimulation patterns. As shown in the example of FIG. 4, timing diagram 400 provides an example of a method for delivering multimodal stimulation, such as differential targeted multiplexed stimulation. Timing diagram 400 shows that pulses 402 can be delivered to two different target tissues, such as the prime pulses 410 in the upper pulse train being delivered to glial cells, as one example. Base pulses 412 in the lower pulse train are less frequent and delivered to a different target tissue, such as neurons associated with the spinal cord. Series of slots 404 indicates that there are four slots that represent a period of time during which a single stimulation pulse can be delivered. Put another way, 4 programs (or respective pulse trains) can be active at the same time, which one pulse from each program being deliverable in its respective slot. The series of slots 404 then continues to repeat over time. In this manner, the pulses of the 4 programs (or respective pulse trains) are at least partially interleaved over time.

In the upper pulse train example of prime pulses 410, the prime stimulation includes pulses delivered during the second, third, and fourth slot of each series of slots 404. The group rate determines the frequency that the series of slots 404 is repeated. Therefore, if the group rate is 300 Hz, prime pulses 410 have a maximum interpulse frequency of 400 Hz and an average of 900 Hz is achieved because the first slot of every series of slots 404 is occupied by the lower train program (the base pulses 412) delivered to a different target tissue via a different electrode combination. As shown in the lower train of base pulses 412 of timing diagram 400, each pulse of the pulse train is only delivered once every sixth occurrence of series of slots 404. When the pulse is not delivered in a series of slots 404, that slot remains empty such that no pulse is delivered. Therefore, the lower train of base pulses 412 achieves a frequency of 50 Hz. In this manner, the system can sense ECAP signals elicited by one pulse of base pulses 412 and prior to the next pulse of base pulses 412. Pattern 406 indicates one complete repeating pattern for the upper and lower trains (prime pulses 410 and base pulses 412) together. As IMD 200 continues to deliver pulses according to the programs and repeating series of slots 404, stimulation is delivered repeatedly with pattern 406 as long as stimulation is being delivered. As discussed herein, at least some of base pulses 412 may be employed as control pulses and at least some pulses of prime pulses 410 may be employed as informed pulses.

In other examples, prime pulse trains and base pulse trains of the multimodal stimulation may be delivered at different frequencies and/or different interleaving schedules than the example of timing diagram 400. For example, prime pulses 410 may be delivered at lower frequencies and/or at different schedules. Prime pulses may still be effective at these lower frequencies and/or different schedules.

The amplitude values for prime pulses 410 and base pulses 412 may be similar or the same in some examples. In other examples, the amplitude value defining prime pulses 410 may be different (e.g., higher or lower) than the amplitude value defining the base pulses 410. One or both of the amplitudes for prime pulses 410 or base pulses 412 may be below a sensory or perception threshold for the patient. For example, stimulation may be initially set so that both of the amplitude values for prime pulses 410 and base pulses 412 are at a predetermined percentage of the perception threshold (e.g., at a value in the range from approximately 50 percent to 90 percent of the perception threshold). The initial percentages may be different for the prime pulses and base pulses. If the patient requires additional stimulation intensity to improve therapy and reduction of symptoms, the patient may provide a user input requesting an adjustment to the stimulation intensity (e.g., amplitude) for the prime and base pulses. IMD 200 may maintain the ratio of the amplitude values of the prime pulses 410 to the base pulses 412. Therefore, IMD 200 may determine the ratio of the amplitude of prime pulses 410 to the amplitude of base pulses 412 and then adjust both amplitudes to different amplitudes that maintain the ratio of amplitudes for prime pulses 410 and base pulses 412. In some examples, one or more of the amplitudes defining prime pulses 410 and base pulses 412 may be above the perception threshold for the patient before or after user adjustments to the amplitude. Although amplitude adjustments are described herein, IMD 200 may maintain the ratio of any values of parameters defining prime pulses 410 and base pulses 412 in response to user input, such as pulse width, pulse frequency, etc.

Although the concept of a series of slots is provided as one example mechanism for managing the delivery of pulses for the first and second stimulation pulses (e.g., the different pulse trains), other management techniques may be used in other examples. For example, IMD 200 may have a flexible programming architecture that enables processing circuitry 210 to schedule different pulses for different electrode combinations at any frequency desired. For example, IMD 200 may simply run multiple different programs that define respective pulse trains interleaved as needed to achieve the respective frequencies of each pulse train. In some examples, one or more pulses of the prime pulse train may be delivered simultaneously with one or more pulses of the base pulse train.

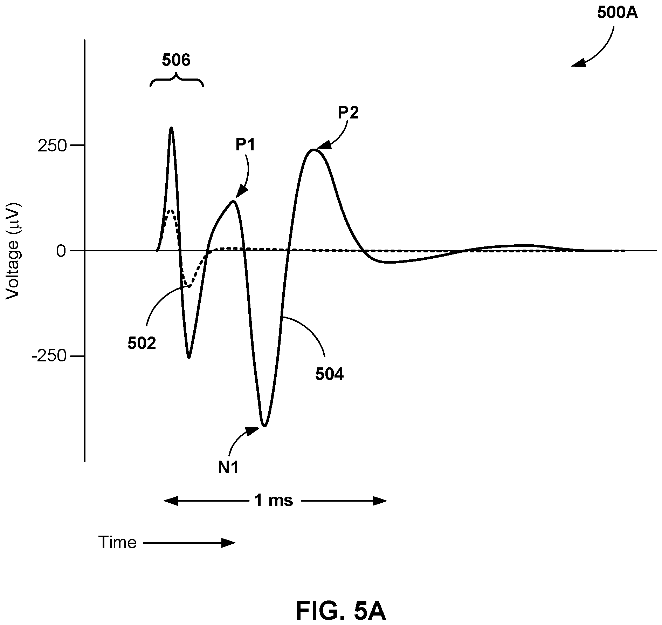

FIG. 5A is a graph 500A of an example EC_AI[3] signals sensed for respective stimulation pulses (e.g., a control pulse). As shown in FIG. 5A, graph 500A shows example ECAI' signal 502 (dotted line) and ECAP signal 504 (solid line). Each of ECAP signals 502 and 504 may be sensed from control pulses that were delivered from a guarded cathode and bi-phasic pulses including an interphase interval between each positive and negative phase of the pulse. The guarded cathode of the stimulation electrodes is located at the end of an 8-electrode lead while two sensing electrodes are provided at the other end of the 8-electrode lead. ECAP signal 502 illustrates the voltage amplitude sensed as a result from a sub-threshold stimulation pulse. Peaks 506 of ECAP signal 502 are detected and represent the artifact of the delivered pulse. However, no propagating signal is detected after the artifact in ECAP signal 504 because the pulse was sub-threshold.

In contrast to ECAP signal 502, ECAP signal 504 represents the voltage amplitude detected from a supra-threshold stimulation pulse. Peaks 506 of ECAP signal 504 are detected and represent the artifact of the delivered pulse. After peaks 506, ECAP signal 504 also includes peaks P1, N1, and P2, which are three peaks representative of propagating action potentials from an ECAP. The example duration of the artifact and peaks P1, N1, and P2 is approximately 1 millisecond (ms). When detecting the ECAP of ECAP signal 504, different characteristics may be identified. For example, the characteristic of the ECAP may be the amplitude between N1 and P2. This N1-P2 amplitude can be detected even if the artifact impinges on P1, a relatively large signal, and the N1-P2 amplitude may be minimally affected by electronic drift in the signal. In other examples, the characteristic of the ECAP used to control pulses may be an amplitude of P1, N1, or P2 with respect to neutral or zero voltage. In some examples, the characteristic of the ECAP used to control pulses may be a sum of two or more of peaks P1, N1, or P2. In other examples, the characteristic of ECAP signal 504 may be the area under one or more of peaks P1, N1, and/or P2. In other examples, the characteristic of the ECAP may be a ratio of one of peaks P1, N1, or P2 to another one of the peaks. In some examples, the characteristic of the ECAP may be a slope between two points in the ECAP signal, such as the slope between N1 and P2. In other examples, the characteristic of the ECAP may be the time between two points of the ECAP, such as the time between N1 and P2. The time between two points in the ECAP signal may be referred to as a latency of the ECAP and may indicate the types of fibers being captured by the pulse. ECAP signals with lower latency (i.e., smaller latency values) indicate a higher percentage of nerve fibers that have faster propagation of signals, whereas ECAP signals with higher latency (i.e., larger latency values) indicate a higher percentage of nerve fibers that have slower propagation of signals. Other characteristics of the ECAP signal may be used in other examples.

The amplitude of the ECAP signal increases with increased amplitude of the pulse, as long as the pulse amplitude is greater than the threshold such that nerves depolarize and propagate the signal. The target ECAP characteristic (e.g., the target ECAP amplitude) may be determined from the ECAP signal detected from a pulse when pulses are determined to deliver effective therapy to the patient. The ECAP signal thus is representative of the distance between the stimulation electrodes and the nerves appropriate for the stimulation parameter values of the pulses delivered at that time. Therefore, IMD 110 may attempt to use detected changes to the measured ECAP characteristic value to change stimulation pulse parameter values and maintain the target ECAP characteristic value during stimulation pulse delivery (e.g., informed pulses and/or control pulses). Alternatively, IMD 110 may attempt to prevent undesirable stimulation intensity by decreasing stimulation pulse intensity in response to the ECAP characteristic value exceeding a threshold ECAP characteristic value.

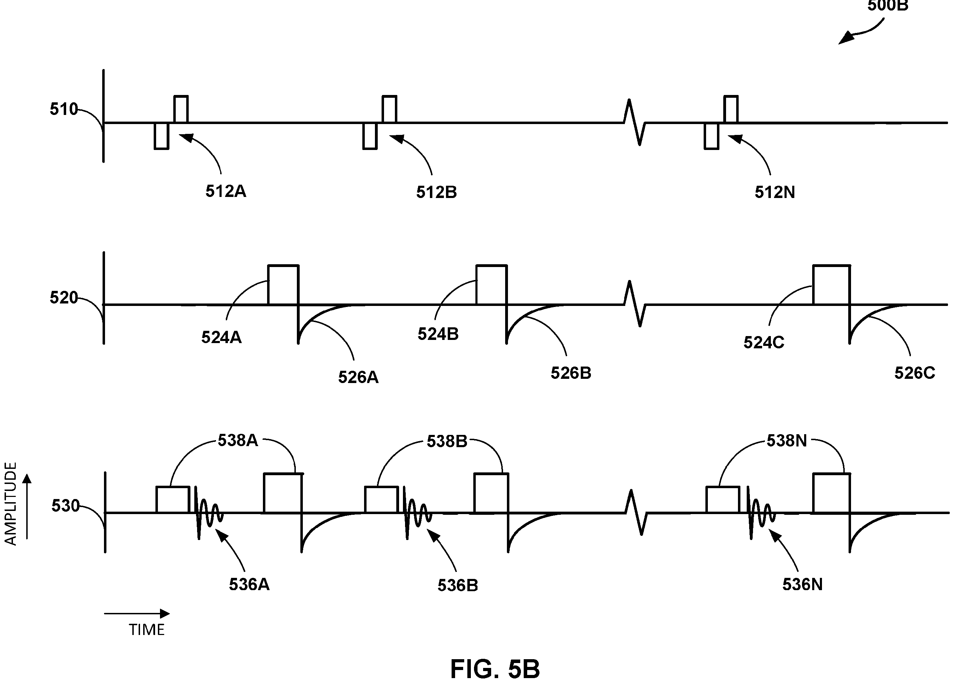

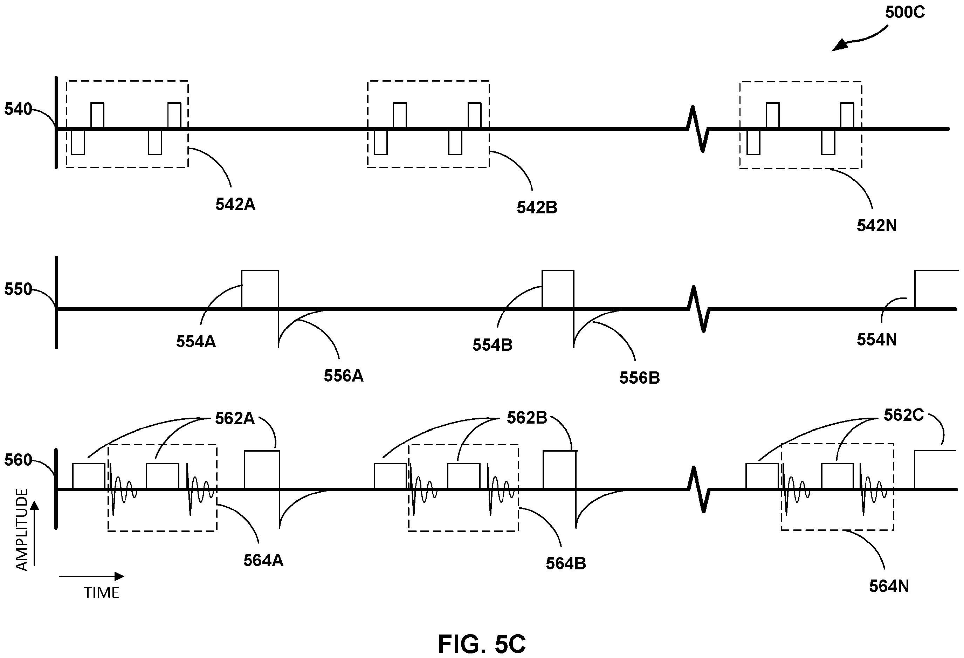

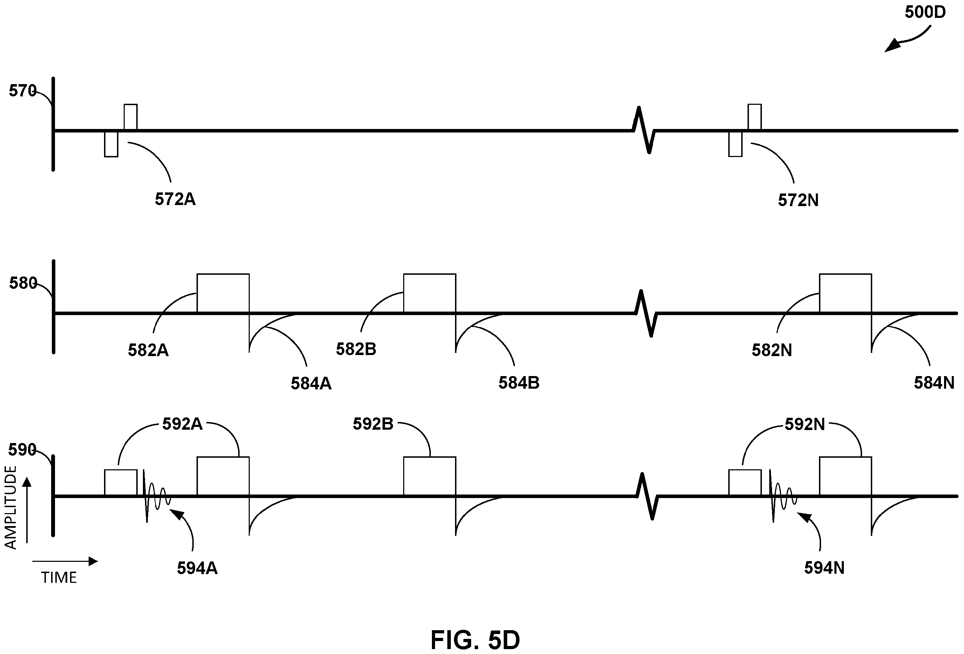

As described in FIGS. 5B, 5C, and 5D, control pulses may include base pulses or tonic pulses from the multimodal stimulation, and informed pulses may include prime pulses from the multimodal stimulation. In this manner, sensed ECAPs elicited by respective base or tonic pulses may inform (e.g., enable the system to control) aspects of stimulation such as one or more parameter values that at least partially defines the prime pulses. FIG. 5B is a timing diagram 500B illustrating another example of electrical stimulation pulses and respective sensed ECAPs, in accordance with one or more techniques of this disclosure. For convenience, FIG. 5B is described with reference to IMD 200 of FIG. 2. As illustrated, timing diagram 500B includes first channel 510, a plurality of control pulses 512A-512N (collectively "control pulses 512"), second channel 520, a plurality of informed pulses 524A-524N (collectively "informed pulses 524") including passive recharge phases 526A-526N (collectively "passive recharge phases 526"), third channel 530, a plurality of respective ECAPs 536A-536N (collectively "ECAPs 536"), and a plurality of stimulation interference signals 538A-538N (collectively "stimulation interference signals 538").

First channel 510 is a time/voltage (and/or current) graph indicating the voltage (or current) of at least one electrode of electrodes 232, 234. In one example, the stimulation electrodes of first channel 510 may be located on the opposite side of the lead as the sensing electrodes of third channel 530. Control pulses 512 may be electrical pulses delivered to the spinal cord of the patient by at least one of electrodes 232, 234, and control pulses 512 may be balanced biphasic square pulses with an interphase interval. In other words, each of control pulses 512 are shown with a negative phase and a positive phase separated by an interphase interval. For example, control pulse 512 may have a negative voltage for the same amount of time and amplitude that it has a positive voltage. It is noted that the negative voltage phase may be before or after the positive voltage phase. Control pulses 512 may be delivered according to control stimulation parameters stored in memory 216 of IMD 200, and the control stimulation parameters may be updated according to user input via an external programmer and/or may be updated according to a signal from sensor(s) 210. As illustrated in FIG. 5B, control pulses 512 may be delivered via channel 510. Delivery of control pulses 512 may be delivered by leads 230 in a guarded cathode electrode combination. For example, if leads 230 are linear 8-electrode leads, a guarded cathode combination is a central cathodic electrode with anodic electrodes immediately adjacent to the cathodic electrode.

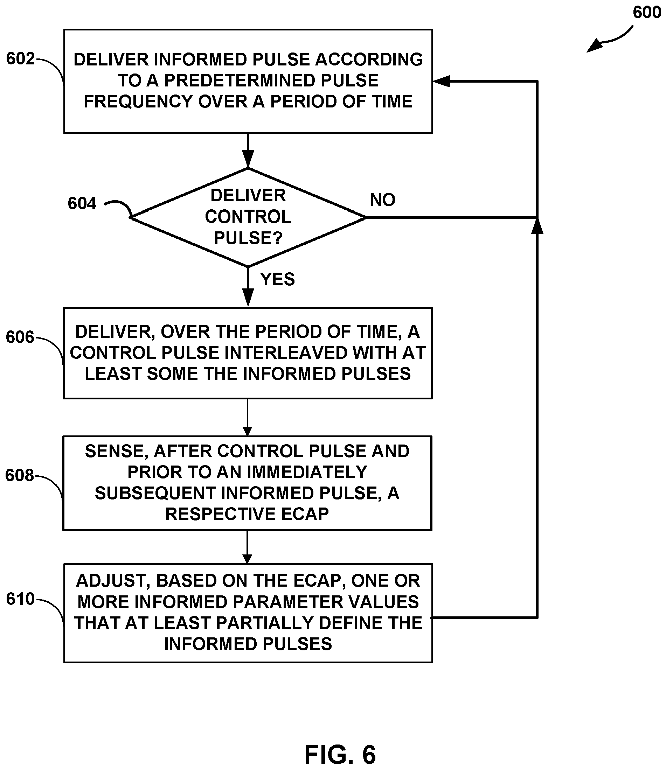

Second channel 520 is a time/voltage (and/or current) graph indicating the voltage (or current) of at least one electrode of electrodes 232, 234 for the informed pulses. In one example, the electrodes of second channel 520 may partially or fully share common electrodes with the electrodes of first channel 510 and third channel 530. Informed pulses 524 may also be delivered by the same leads 230 that are configured to deliver control pulses 512. The amplitude of informed pulses 524 may be different from the amplitude of control pulses 512. Informed pulses 524 may be interleaved with control pulses 512, such that the two types of pulses are not delivered during overlapping periods of time. However, informed pulses 524 may or may not be delivered by exactly the same electrodes that deliver control pulses 512. As illustrated in FIG. 6B, informed pulses 524 may be delivered on channel 520.

Informed pulses 524 may be configured for passive recharge. For example, each informed pulse 524 may be followed by a passive recharge phase 526 to equalize charge on the stimulation electrodes. Unlike a pulse configured for active recharge, wherein remaining charge on the tissue following a stimulation pulse is instantly removed from the tissue by an opposite applied charge, passive recharge allows tissue to naturally discharge to some reference voltage (e.g., ground or a rail voltage) following the termination of informed pulse 524. In some examples, the electrodes of the medical device may be grounded at the medical device body. In this case, following the termination of informed pulse 524, the charge on the tissue surrounding the electrodes may dissipate to the medical device, creating a rapid decay of the remaining charge at the tissue following the termination of the pulse. This rapid decay is illustrated in passive recharge phases 526. Passive recharge phase 526 may have a duration in addition to the pulse width of the preceding informed pulse 524. In other examples (not pictured in FIG. 5B), informed pulses 524 may be bi-phasic pulses having a positive and negative phase (and, in some examples, an interphase interval between each phase) which may be referred to as pulses including active recharge. Informed pulse 524 that is a bi-phasic pulse may or may not have a following passive recharge phase.

Third channel 530 is a time/voltage (and/or current) graph indicating the voltage (or current) of at least one electrode of electrodes 232, 234. In one example, the electrodes of third channel 530 may be located on the opposite side of the lead as the electrodes of first channel 510. ECAPs 536 may be sensed at electrodes 232, 234 from the spinal cord of the patient in response to control pulses 512. ECAPs 536 are electrical signals which may propagate along a nerve away from the origination of control pulses 512. In one example, ECAPs 536 are sensed by different electrodes than the electrodes used to deliver control pulses 512. As illustrated in FIG. 5B, ECAPs 536 may be recorded on third channel 530.

Stimulation interference signals 538A, 538B, and 538N (e.g., the artifact of the stimulation pulses) may be sensed by leads 230 and may be sensed during the same period of time as the delivery of control pulses 512 and informed pulses 524. Since the interference signals may have a greater amplitude and intensity than ECAPs 536, any ECAPs arriving at IMD 200 during the occurrence of stimulation interference signals 538 may not be adequately sensed by sensing circuitry 206 of IMD 200. However, ECAPs 536 may be sufficiently sensed by sensing circuitry 206 because each ECAP 536 falls after the completion of each control pulse 512 and before the delivery of the next informed pulse 524. As illustrated in FIG. 5B, stimulation interference signals 538 and ECAPs 536 may be recorded on channel 530.

Two or more control pulses 512 may be delivered during each time event (e.g., window) of a plurality of time events, and each time event represents a time between two consecutive informed pulses 524. For example, during each time event, a first control pulse may be directly followed by a first respective ECAP, and subsequent to the completion of the first respective ECAP, a second control pulse may be directly followed by a second respective ECAP. Informed pulses may commence following the second respective ECAP.

Consecutive informed pulses 524 may be delivered without intervening control pulse 512. For example, control pulses 512 may not be delivered during each time event (or window) of the plurality of time events, wherein each time event represents a time between two consecutive informed pulses 524. In any case, informed pulses 524 can be delivered according to a predetermined frequency, and control pulses 512 may be delivered at any time between the informed pulses.

FIG. 5C is a timing diagram 500C illustrating another example of electrical stimulation pulses and respective ECAPs according to the techniques of the disclosure. For convenience, FIG. 5C is described with reference to IMD 200 of FIG. 2A. As illustrated, timing diagram 500C includes first channel 540, a plurality of control pulses 542A-542N (collectively "control pulses 542"), second channel 550, a plurality of informed pulses 554A-554N (collectively "informed pulses 554") including passive recharge phases 556A-556N (collectively "passive recharge phases 556"), third channel 560, a plurality of respective ECAPs 564A-564N (collectively "ECAPs 564"), and a plurality of stimulation interference signals 562A-562N (collectively "stimulation interference signals 562"). FIG. 5C may be substantially similar to FIG. 5B, except for the differences detailed below.

Two or more (e.g. two) control pulses 542 may be delivered during each time event (e.g., window) of a plurality of time events, and each time event represents a time between two consecutive informed pulses 554. For example, during each time event, a first control pulse of control pulses 542A may be directly followed by a first respective ECAP of ECAPs 564A, and subsequent to the completion of the first respective ECAP, a second control pulse of control pulses 542A may be directly followed by a second respective ECAP of ECAPs 564A. Informed pulses 554 may commence following the second respective ECAP. In other examples not illustrated here, three or more control pulses 542 may be delivered, and respective ECAP signals sensed, during each time event of the plurality of time events.

FIG. 5D is a timing diagram 500D illustrating another example of electrical stimulation pulses and respective ECAPs according to the techniques of the disclosure. For convenience, FIG. 5D is described with reference to IMD 200 of FIG. 2A. As illustrated, timing diagram 500D includes first channel 570, a plurality of control pulses 572A-572N (collectively "control pulses 572"), second channel 580, a plurality of informed pulses 582A-582N (collectively "informed pulses 582") including passive recharge phases 584A-584N (collectively "passive recharge phases 584"), third channel 590, a plurality of respective ECAPs 594A-594N (collectively "ECAPs 594"), and a plurality of stimulation interference signals 592A-592N (collectively "stimulation interference signals 592"). FIG. 6 may be substantially similar to FIG. 5B and 5C, except for the differences detailed below.

In previous examples illustrated in FIG. 5B and FIG. 5C, at least one control pulse was delivered and interleaved between each pair of consecutive informed pulses. However, in some examples, control pulses 572 are not delivered during each time event (or window) of the plurality of time events, wherein each time event represents a time between two consecutive informed pulses 582. As illustrated in the example of FIG. 5D, a control pulse 572 is not delivered following informed pulse 582A and preceding informed pulse 582B. In other words, consecutive informed pulses 582A and 582B may be delivered without an intervening control pulse. This scenario may be more similar to the timing diagram 400 in FIG. 4 wherein many prime pulses are delivered between consecutive base pulses. In any case, informed pulses are delivered according to a predetermined frequency, and control pulses may be delivered at any time between the informed pulses. In some examples, the parameter values of both informed pulse 582A and informed pulse 582B may be the same because they are defined by the same stimulation program. In other examples, informed pulse 582A and informed pulse 582B may have at least one stimulation parameter that differs in value, such as a different amplitude, pulse width, pulse frequency, or electrode combination. in this manner, informed pulse 582A may be a part of a first stimulation program while informed pulse 582B may be part of a second stimulation program that is different than the first stimulation program. Processing circuitry 214 may thus delivery informed pulses from two or more different stimulation programs, where processing circuitry 214 uses the detected ECAP signal from the same control pulse (e.g., control pulse 572A) to "inform" or otherwise adjust one or more parameter values of the informed pulses in multiple stimulation programs (e.g., both of informed pulses 582A and 582B). This concept of multiple stimulation programs may be applied to any informed pulses described herein.

Control pulses 572 may be administered according to control stimulation parameters stored in memory 216. Processing circuitry 214 may be configured to update the control pulse delivery instructions according to user input via telemetry circuitry 213, and also by a signal from sensor 216. For example, a clinician may operate a patient programmer and send a signal to telemetry circuitry 213 including instructions for updating the control pulse parameters. The clinician may set control stimulation to any of the examples illustrated in FIGS. 5B-5D, and the clinician also may customize control stimulation to a configuration not illustrated in FIGS. 5B-5D. The clinician may elect to cease control stimulation or commence control stimulation at any time. In some examples, a detection that the patient's posture or activity level has changed will initiate control stimulation. Although the control pulses illustrated in the examples of FIGS. 5B-5D are described as bi-phasic pulses that include an active recharge phase as the second phase of the bi-phasic pulse, one or more control pulses may include a passive recharge phase as the second phase of the control pulse in other examples.

FIG. 6 is a flowchart illustrating an example operation 600 for informed delivery based on sensed ECAP signals. For convenience, FIG. 6 is described with respect to processing circuitry 208 of IMD 200 of FIG. 2 and processing circuitry 302 of FIG. 3. However, the techniques of FIG. 6 may be performed by different components of IMD 200, programmer 300, or by additional or alternative medical devices in some examples.

In the example of FIG. 6, IMD 200 delivers electrical stimulation therapy to patient 102, the electrical stimulation therapy comprising at least one informed pulse at a predetermined pulse frequency over a period of time (602). If processing circuitry 208 determines not to deliver a control pulse ("NO" branch of block 604), processing circuitry 208 continues to deliver informed pulses (602). In this example, each informed pulse may be a prime pulse of one or more prime pulse trains, and each control pulse may be a base pulse of a base pulse train. If processing circuitry 208 determines to deliver a control pulse ("YES" branch of block 604), processing circuitry 208 delivers, over the period of time, a control pulse interleaved with at least some informed pulses of the plurality of informed pulses (606). IMD 200 may sense, after each control pulse and prior to an immediately subsequent informed pulse of the plurality of informed pulses, a respective ECAP signal (608). In some examples, IMD 200 may sense the ECAP signal at least partially concurrently in time with delivery of an informed pulse.

IMD 200 may determine an ECAP characteristic value from the ECAP signal that represents the ECAP signal. For example, IMD 200 may determine the characteristic value to include at least one of an ECAP amplitude between two peaks of the ECAP signal, an area under a curve of at least a portion of the ECAP signal, a latency of at least one feature of the ECAP signal, a spectral content of the ECAP signal, a presence of one or more features of the ECAP signal, an absence of one or more features of the ECAP signal, or a combination of at least one peak and at least one trough of the ECAP signal. Processing circuitry 208 may compare this ECAP characteristic value to a target ECAP value or some other value.

Subsequent to the sensing, IMD 200 may adjust, based on at least one respective ECAP signal or ECAP characteristic value, one or more informed parameter values that at least partially define the plurality of informed pulses of the electrical stimulation therapy (610). For example, processing circuitry 208 may adjust an amplitude that defines subsequent pulses of the prime pulse train (e.g., the informed pulses). Processing circuitry 208 may then continue to deliver informed pulses using the new parameter value (602).

In other examples, processing circuitry 208 may adjust other parameters than amplitude. For example, processing circuitry 208 may adjust an electrode combination of the prime pulse train of electrical stimulation pulses, a number of pulses in the prime pulse train during a duty cycle, the first frequency of pulses in the prime pulse train, an amplitude of pulses in the prime pulse train, a pulse width of pulses in the prime pulse train, a frequency modulation factor that modulates the first frequency of the prime pulse train, an amplitude modulation factor that modulates the amplitude of the pulses in the prime pulse train, an interphase interval of the pulses in the prime pulse train, a pulse shape of the pulses in the prime pulse train, and/or a polarity of electrodes of the prime pulse train. Processing circuity 208 may use instructions for predetermined adjustments to make based on ECAP values, or processing circuity 208 may being testing different parameter values in response to the ECAP value migrating from a target ECAP value. For example, processing circuity 208 may begin trying different nearby electrode combinations for the prime pulse train if the previous electrode combination does not result in achieving the target ECAP value. In addition, or alternatively, processing circuitry 208 may adjust similar parameters of the base pulse train. In some examples, processing circuitry 208 may be maintain similar ratios between the parameter for the prime pulse train and the base pulse train when adjusting the values of the parameter for both pulse trains.

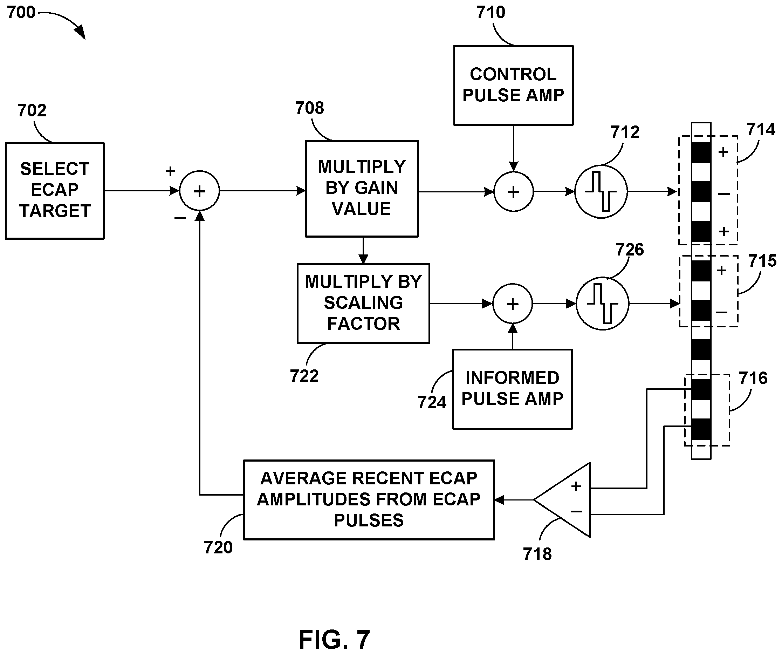

FIG. 7 is a diagram illustrating an example technique 700 for adjusting stimulation therapy. As shown in the example of FIG. 7, the system, such as IMD 200 or any other device or system described herein, may dynamically adjust pulse amplitude (or other parameter) based on the gain value representing the patient sensitivity to stimulation. Processing circuitry 208 of IMD 200 may control stimulation generator 204 to deliver a stimulation pulses, such as informed pulses (e.g., pulses of one or more prime pulse trains) and control pulses (e.g., pulses of a base pulse train), to a patient Processing circuitry 208 may then control sensing circuitry 206 to sense an ECAP signal elicited by the control pulse and then identify a characteristic of the ECAP signal (e.g., an amplitude of the ECAP signal). Processing circuitry 208 may then determine, based on the characteristic of the ECAP signal and a gain value, a parameter value (e.g., an amplitude, pulse width value, pulse frequency value, and/or slew rate value) that at least partially defines stimulation pulses, such as control pulses and informed pulses. Processing circuitry 208 may then control stimulation generator 204 to deliver the informed pulses and control pulses according to the determined stimulation parameters.

As shown in FIG. 7, control pulse 712 is a pulse of the base pulse train and is delivered to the patient via electrode combination 714, shown as a guarded cathode of three electrodes. The resulting ECAP is sensed by the two electrodes at the opposing end of the lead of electrode combination 716 fed to a differential amplifier 718. For each sensed ECAP, processing circuitry 208 may measure an amplitude of a portion of the ECAP signal, such as the N1-P2 voltage amplitude from the portion of the ECAP signal. Processing circuitry 208 may average the recently measured ECAP amplitudes, such as averaging the most recent, and consecutive, 2, 3, 4, 5, 6, or more ECAP amplitudes. In some examples, the average may be a mean or median value. In some examples, one or more ECAP amplitudes may be ignored from the calculations if the amplitude value is determined to be an error. The measured amplitude (or average measured amplitude) is then subtracted from the selected target ECAP amplitude 702 to generate a differential amplitude. The selected target ECAP amplitude 702 may be determined from an ECAP sensed when the physician or patient initially discovers effective therapy from the informed pulses and/or control pulses. This target ECAP amplitude 702 may essentially represent a reference distance between the stimulation electrodes and the target neurons (e.g., the spinal cord for the case of SCS).

The differential amplitude is then multiplied by the gain value for the patient to generate a differential value 708. Processing circuitry 208 may add the differential value to the ECAP pulse amplitude to generate the new, or adjusted, control pulse amplitude 710 that at least partially defines the next pulse 712.

The following formulas may represent the function used to calculate the pulse amplitude of the next pulse 712, Equation 1 below represents an equation for calculating the new current amplitude using a linear function, wherein Ac is the current pulse amplitude, D is the differential amplitude by subtracting the measured amplitude from the target ECAP amplitude, G is a real number for the gain value, and $A_N$ is the new pulse amplitude:

$$A_N = A_C + (D \times G) \tag{1}$$

In this manner, the gain value G may not change for a given input. Alternatively, processing circuitry 208 may calculate the gain value G such that the gain value varies according to one or more inputs or factors, such as posture state. In this manner, for a given input or set of inputs, processing circuitry 208 may change the gain value G. Equation 2 below represents an example linear function for calculating the gain value, wherein M is a multiplier, D is the differential amplitude by subtracting the measured amplitude from the target ECAP amplitude, and G is the gain value:

$$G = M \times D \qquad\qquad (2)$$

Processing circuitry 208 may use the gain value G calculated in Equation 2 in Equation 1. This would result in Equation 1 being a non-linear function for determining the new current amplitude. According to Equation 2 above, the gain value G may be greater for larger differences between the measured amplitude and the target ECAP amplitude. Thus, gain value G will cause non-linear changes to the current amplitude. In this manner, the rate of change in the current amplitude will be higher for larger differences between the measured amplitude and the target ECAP amplitude and lower for smaller differences between the measured amplitude and the target ECAP amplitude. In other examples, a non-linear function may be used to calculate the gain value G.

To adjust the informed pulse amplitude, the differential value 708 is multiplied by a scaling factor 722 to generate the informed differential value. For example, the scaling factor may be the ratio of the previously delivered informed pulse amplitude to the previously delivered control pulse amplitude. This scaling factor may be similar to the ratio between the parameter values of the control pulses and informed pulses used in response to user input adjusting stimulation parameters. The informed differential value is then added to the previously delivered informed pulse amplitude 724 to generate the new, or adjusted, informed pulse amplitude that at least partially defines the next one or more informed pulse 726. The next informed pulse 726 is then delivered, interleaved with control pulse 712, to the patient via electrode combination 715. In some examples, many informed pulses 726 may be delivered before the next control pulse 712 is delivered. In some examples, at least two control pulses may be delivered, and at least two respective ECAP signals sensed, between consecutive informed pulses. This increased frequency of non-therapeutic pulses may allow the system to quickly adjust informed pulse amplitudes for any changes in the distance between electrodes and neurons. Although electrode combination 715 is different than electrode combinations 714 and 716, electrode combination 715 can be any set of electrodes on the lead as desired for therapy because the informed pulse is delivered in a non-overlapping fashion with control pulses and sensed ECAP signals. Also, electrode combination 715 may include electrodes from the same lead or different lead(s) than the electrodes of electrode combination 714.

In some examples, depending upon, at least in part, pulse width of the control pulse, IMD 200 may not sufficiently detect an ECAP signal because the stimulation pulse is also detected as an artifact that obscures the ECAP signal. If ECAPs are not adequately recorded, then ECAPs arriving at IMD 200 cannot be used to determine the efficacy of stimulation parameter settings, and electrical stimulation signals cannot be altered according to responsive ECAPs. In some examples, pulse widths of the control pulses may be less than approximately 300 μs, which may increase the number of ECAP signals detected. Similarly, high pulse frequencies (such as a pulse frequency of one or more prime pulse trains) may interfere with IMD 200 sufficiently detecting ECAP signals. For example, at pulse frequency values (e.g., greater than 1 kHz) that cause IMD 200 to deliver another control pulse before an ECAP from the previous pulse can be detected, IMD 200 may not be capable to detecting the ECAP.

In some examples, IMD 200 may adjust other parameters associated with stimulation or closed-loop control of multimodal stimulation. For example, IMD 200 may adjust, in response to and based on an ECAP signal or characteristic value, a gain value that at least partially determines adjustment of one or more parameters of stimulation, one or more filtering characteristics of the ECAP signal, or a sensing electrode combination (e.g., electrode combination 716) used to sense the ECAP signal.

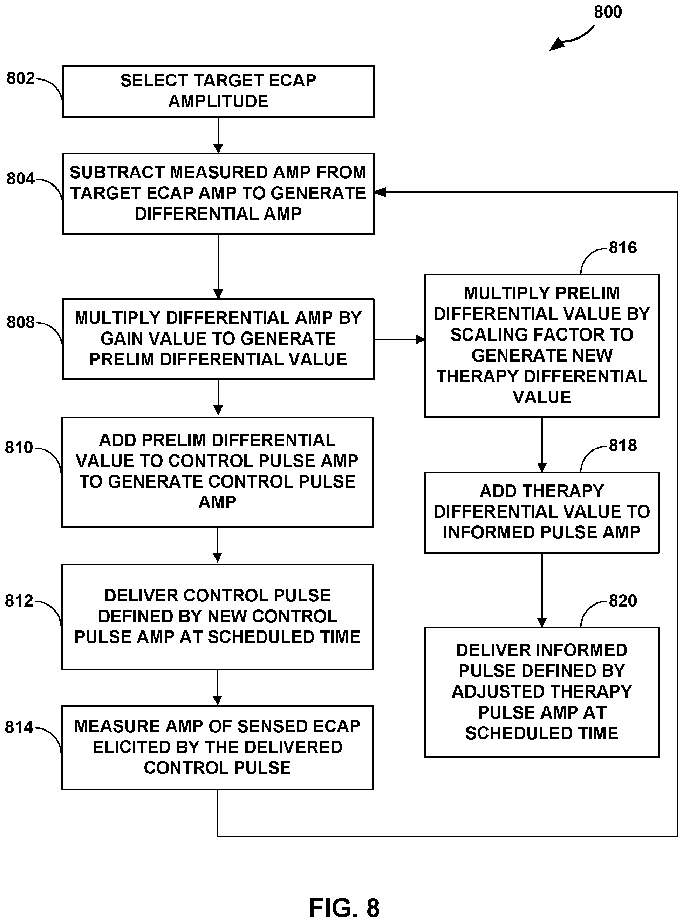

FIG. 8 is a flowchart illustrating an example operation 800 for adjusting stimulation parameters, such as informed parameters and control parameters (e.g., prime pulse parameters and base pulse parameters, respectively). IMD 200 and processing circuitry 208 will be described in the example of FIG. 8, but other IMDs such as IMD 110 or other devices or systems may perform, or partially perform, operation 800. Operation 800 may be similar to the diagram and discussion related to FIG. 7.

In the example of FIG. 8, processing circuitry 208 determines the target ECAP amplitude (802). The target ECAP amplitude may be determined based on sample stimulation initially delivered to the patient. The target ECAP amplitude may be the N1-P2 amplitude of the ECAP signal, but other measures of amplitude, such as amplitude of one or more different peaks in the ECAP signal may be used instead. Alternatively, the target ECAP amplitude may instead be a different characteristic of the ECAP signal such as the area under one or more peaks of the ECAP signal. In some examples, processing circuitry 208 is configured to automatically change the target ECAP amplitude over a period of time according to a predetermined function (e.g., a sinusoid function) in order to change the volume of neuron activation and, in some examples, the perceived sensation of the informed pulses.

Processing circuitry 208 receives a measured amplitude from the previously sensed ECAP signal. In order to use the ECAP signal as feedback to control the informed pulses (e.g., pulses of the prime pulse train) of electrical stimulation therapy for the patient, processing circuitry 208 subtracts the measured amplitude from the target ECAP amplitude to generate a differential amplitude (804). In some examples, or as additional measured amplitudes are available from the process, processing circuitry 208 may average a certain number of recent measured amplitudes (e.g., two or more) to create a rolling average of measured ECAP amplitudes and subtract the average measured amplitudes from the target ECAP amplitude to smooth out variations between ECAP signals. The differential amplitude is thus a representation of how far of a distance the electrodes have moved relative to the neurons and can be used to adjust the amplitudes of the informed pulses and the control pulses to maintain consistent volume of neural activation of the neurons that provide relief to the patient.

Processing circuitry 208 then multiplies the differential amplitude from block 904 by a gain value to generate a preliminary differential value (808). The gain value may represent the slope of the growth curve for the patient. Processing circuitry 208 then uses the preliminary differential value to adjust the amplitudes of both subsequent informed pulses and control pulses (e.g., pulses of the base pulse tram). Processing circuitry 208 adds the preliminary differential value to the control pulse amplitude to generate a new control pulse amplitude (810). Processing circuitry 208 then controls stimulation generation circuitry 208 to deliver a subsequent control pulse defined by the new control pulse amplitude at a scheduled time, such as according to the frequency of the control pulses or according to the next available window between informed pulses (812). Processing circuitry 208 also controls sensing circuitry 206 to measure the amplitude of the sensed ECAP elicited by the recently delivered control pulse (814) to use again as feedback in block 804.

In addition to adjusting the amplitude of the control pulses, processing circuitry 208 uses the preliminary amplitude to adjust the informed pulse amplitude. Processing circuitry 208 multiplies the differential value by a scaling factor to generate a new informed differential value (816). The scaling factor may be determined as the ratio between the amplitude of the most recently delivered informed pulse and the amplitude of the most recently delivered control pulse that elicited the ECAP signal used to generate the measured amplitude used in block 804. The scaling factor may scale up, or scale down, the differential amplitude for the informed pulses because the differential amplitude was generated based on amplitudes of control pulses. Processing circuitry 208 then adds the therapy differential value to the most recent informed pulse amplitude to generate a new informed pulse amplitude (818). Processing circuitry 208 then controls stimulation generator 204 to deliver the next informed pulse with the newly adjusted informed pulse amplitude at the scheduled time according to the predetermined pulse frequency of the informed pulses (820).

Although operation 800 is described for adjusting the amplitude of informed pulses and control pulses, a similar operation may be used to adjust other stimulation parameters in other examples. For example, parameters that contribute to the intensity of the informed pulses and control pulses may affect the volume of neural activation, such parameters as pulse width, pulse frequency, or even pulse shape (e.g., the amount of charge per pulse). Therefore, processing circuitry 208 can adjust a different parameter instead of, or in addition to, amplitude using the sensed ECAP signal elicited from the control pulses. For example, processing circuitry 208 may increase the pulse width of the informed pulses and control pulses in response to detecting a decreased ECAP amplitude. In addition, processing circuitry 208 can adjust parameters based on a change of patient posture state as well as a different growth curve being selected.

The techniques described in this disclosure may be implemented, at least in part, in hardware, software, firmware or any combination thereof. For example, various aspects of the described techniques may be implemented within one or more processors or processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit including hardware may also perform one or more of the techniques of this disclosure,

Such hardware, software, and firmware may be implemented within the same device or within separate devices to support the various operations and functions described in this disclosure. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components.

The techniques described in this disclosure may also be embodied or encoded in a computer-readable medium, such as a computer-readable storage medium, containing instructions that may be described as non-transitory media. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

**Claims**

1. A system comprising:
   processing circuitry (208) configured to:

   control delivery of a first train of electrical stimulation pulses at a first frequency to a first target tissue;
   control delivery of a second train of electrical stimulation pulses at a second frequency to a second target tissue different from the first target tissue, wherein at least some electrical stimulation pulses of the first train of electrical stimulation pulses are interleaved with at least some electrical stimulation pulses of the second train of electrical stimulation pulses, and wherein the first frequency is greater than the second frequency;
   receive an evoked compound action potential (ECAP) signal elicited by a pulse of the second train of electrical stimulation pulses;
   adjust, based on the ECAP signal, a first value of a parameter that at least partially defines the first train of electrical stimulation pulses to a second value; and
   responsive to adjusting the first value of the parameter to the second value, control delivery of subsequent pulses of the first train of electrical stimulation pulses according to the second value of the parameter.

2. The system of claim 1, further comprising stimulation generation circuitry configured to deliver the first train of electrical stimulation pulses and the second train of electrical stimulation pulses, and wherein the processing circuitry (208) is configured to control the stimulation generation circuitry (204) to deliver the first train of electrical stimulation pulses and the second train of electrical stimulation pulses.

3. The system of any of claims 1 and 2, wherein the parameter comprises one of:

   an electrode combination of the first train of electrical stimulation pulses;
   a number of pulses in the first train during a duty cycle;
   the first frequency of pulses in the first train;
   an amplitude of pulses in the first train;
   a pulse width of pulses in the first train;
   a frequency modulation factor that modulates the first frequency of the first train;
   an amplitude modulation factor that modulates the amplitude of the pulses in the first train;
   an interphase interval of the pulses in the first train;
   a pulse shape of the pulses in the first train; or
   a polarity of electrodes of the first train.

4. The system of any of claims 1 through 3, further comprising sensing circuitry configured to sense the ECAP signal elicited by the pulse of the second train of electrical stimulation pulses.

5. The system of claim 4, wherein the processing circuitry (208) is configured to determine, from the ECAP signal, a characteristic value which is an ECAP amplitude of a portion of the ECAP signal, wherein the parameter comprises a first train amplitude of pulses of the first train, wherein pulses of the second train comprise a second train amplitude, and wherein the processing circuitry (208) is further configured to adjust the first value of the parameter to the second value of the parameter by at least:

   subtracting the ECAP amplitude from a target ECAP amplitude value for the patient to generate a differential amplitude;
   multiplying the differential amplitude by a gain value to generate a preliminary differential value;
   multiplying the preliminary differential value by a scaling factor to generate an informed differential value, wherein the scaling factor represents the ratio;
   adding the informed differential value to the first value of first train amplitude to generate the second value of the first train amplitude; and
   adding the preliminary differential value to the first value of the second train amplitude to generate a second value of the second train amplitude for subsequent pulses of the second train.

6. The system of any of claims 1 through 5, wherein the processing circuitry (208):

determines a characteristic value of the ECAP signal; and
adjusts, based on the characteristic value, the first value of the parameter that at least partially defines the first train of electrical stimulation pulses to the second value,
wherein the characteristic value comprises:

an ECAP amplitude between two peaks of the ECAP signal;
an area under a curve of at least a portion of the ECAP signal;
a latency of at least one feature of the ECAP signal;
a spectral content of the ECAP signal;
a presence of one or more features of the ECAP signal;
an absence of one or more features of the ECAP signal; or
a combination of at least one peak and at least one trough of the ECAP signal.

7. The system of any of claims 1 through 6, wherein the processing circuitry (208) is further configured to adjust, based on the ECAP signal, at least one of:

a gain value that at least partially determines adjustment of the parameter;
one or more filtering characteristics of the ECAP signal; or
a sensing electrode combination used to sense the ECAP signal.

8. The system of any of claims 1 through 7, wherein the first train of electrical stimulation pulses comprises two or more pulse trains that have an average frequency less than the first frequency and greater than the second frequency.

9. The system of any of claim 8, wherein the average frequency is selected from a frequency range from 150 Hz to 900 Hz.

10. The system of any of claims 1 through 9, wherein the first target tissue comprises a first type of cells and the second target tissue comprises a second type of cells different from the first type of cells.

11. The system of any of claims 1 through 10, wherein the processing circuitry (208) is configured to control delivery the first train and deliver the second train by at least controlling delivery of the first train and the second train of electrical stimulation pulses in a repeatable series of slots, the repeatable series of slots being repeatable over time for delivery of the first train of electrical stimulation pulses and the second train of electrical stimulation pulses, and wherein:

delivery of the first train of electrical stimulation pulses comprises generating one pulse for a first slot of at least some of the repeatable series of slots that achieves the first frequency, and
delivery of the second train of electrical stimulation pulses comprises generating one pulse for a second slot of at least some of the repeatable series of slots that achieves the second frequency.

12. The system of any of claims 1 through 11, wherein the second frequency is selected from a frequency range from 40 Hz to 60 Hz.

13. The system of any of claims 1 through 12, wherein the parameter comprises an amplitude, and wherein the first value of the amplitude that at least partially defines the first train of electrical stimulation pulses is below at least one of a perception threshold or a sensory threshold of a patient.

14. The system of any of claims 1 through 13, further comprising an implantable medical device comprising the processing circuitry (208).

15. The system of any of claims 1 through 14, wherein the first target tissue comprises glial cells, and wherein the second target tissue comprises neurons.

**Patentansprüche**

1. System, umfassend:

eine Verarbeitungsschaltungsanordnung (208), die ausgelegt ist zum:

Steuern der Verabreichung einer ersten Folge elektrischer Stimulationsimpulse mit einer ersten Frequenz an ein erstes Zielgewebe;

Steuern der Verabreichung einer zweiten Folge elektrischer Stimulationsimpulse mit einer zweiten Frequenz an ein zweites Zielgewebe, das sich von dem ersten Zielgewebe unterscheidet, wobei zumindest einige elektrische Stimulationsimpulse der ersten Folge elektrischer Stimulationsimpulse mit zumindest einigen elektrischen Stimulationsimpulsen der zweiten Folge elektrischer Stimulationsimpulse verschachtelt sind und wobei die erste Frequenz größer als die zweite Frequenz ist;

Empfangen eines Signals eines evozierten zusammengesetzten Aktionspotentials (ECAP), das durch einen Impuls der zweiten Folge elektrischer Stimulationsimpulse ausgelöst wird;

Einstellen eines ersten Wertes eines Parameters, der zumindest teilweise die erste Folge elektrischer Stimulationsimpulse definiert, basierend auf dem ECAP-Signal auf einen zweiten Wert; und

in Reaktion auf das Einstellen des ersten Wertes des Parameters auf den zweiten Wert Steuern der Verabreichung der nachfolgenden Impulse der ersten Folge elektrischer Stimulationsimpulse gemäß dem zweiten Wert des Parameters.

2. System nach Anspruch 1, ferner umfassend eine Stimulationserzeugungsschaltungsanordnung, die zum Verabreichen der ersten Folge elektrischer Stimulationsimpulse und der zweiten Folge elektrischer Stimulationsimpulse ausgelegt ist, und wobei die Verarbeitungsschaltungsanordnung (208) so ausgelegt ist, dass sie die Stimulationserzeugungsschaltungsanordnung (204) zum Verabreichen der ersten Folge elektrischer Stimulationsimpulse und der zweiten Folge elektrischer Stimulationsimpulse steuert.

3. System nach einem der Ansprüche 1 und 2, wobei der Parameter eines von Folgenden umfasst:

eine Elektrodenkombination der ersten Folge elektrischer Stimulationsimpulse;
eine Anzahl von Impulsen in der ersten Folge während eines Arbeitszyklus;
die erste Frequenz von Impulsen in der ersten Folge;
eine Amplitude von Impulsen in der ersten Folge;
eine Impulsbreite von Impulsen in der ersten Folge;
einen Frequenzmodulationsfaktor, der die erste Frequenz der ersten Folge moduliert;
einen Amplitudenmodulationsfaktor, der die Amplitude der Impulse in der ersten Folge moduliert;
ein Zwischenphasenintervall der Impulse in der ersten Folge;
eine Impulsform der Impulse in der ersten Folge oder eine Polarität von Elektroden der ersten Folge.

4. System nach einem der Ansprüche 1 bis 3, ferner umfassend eine Sensorschaltungsanordnung, die so ausgelegt ist, dass sie das ECAP-Signal erfasst, das durch den Impuls der zweiten Folge elektrischer Stimulationsimpulse ausgelöst wird.

5. System nach Anspruch 4, wobei die Verarbeitungsschaltungsanordnung (208) so ausgelegt ist, dass sie aus dem ECAP-Signal einen charakteristischen Wert bestimmt, der eine ECAP-Amplitude eines Abschnitts des ECAP-Signals ist, wobei der Parameter eine Erste-Folge-Impulsamplitude der ersten Folge umfasst, wobei Impulse der zweiten Folge eine Amplitude der zweiten Folge umfassen, und wobei die Verarbeitungsschaltungsanordnung (208) ferner so ausgelegt ist, dass sie den ersten Wert des Parameters zumindest durch Folgendes auf den zweiten Wert des Parameters einstellt:

Subtrahieren der ECAP-Amplitude von einem Ziel-ECAP-Amplitudenwert für den Patienten, um eine Differenzamplitude zu erzeugen;

Multiplizieren der Differenzamplitude mit einem Verstärkungswert, um einen vorläufigen Differenzwert zu erzeugen;

Multiplizieren des vorläufigen Differenzwerts mit einem Skalierungsfaktor, um einen fundierten Differenzwert zu erzeugen, wobei der Skalierungsfaktor das Verhältnis darstellt;

Addieren des fundierten Differenzwerts zu dem ersten Wert der Amplitude der ersten Folge, um den zweiten Wert der Amplitude der ersten Folge zu erzeugen, und

Addieren des vorläufigen Differenzwerts zu dem ersten Wert der Amplitude der zweiten Folge, um einen zweiten Wert der Amplitude der zweiten Folge für nachfolgende Impulse der zweiten Folge zu erzeugen.

6. System nach einem der Ansprüche 1 bis 5, wobei die Verarbeitungsschaltungsanordnung (208):

einen charakteristischen Wert des ECAP-Signals bestimmt; und
den ersten Wert des Parameters, der zumindest teilweise die erste Folge elektrischer Stimulationsimpulse definiert, basierend auf dem charakteristischen Wert auf den zweiten Wert einstellt, wobei der charakteristische Wert Folgendes umfasst:

eine ECAP-Amplitude zwischen zwei Spitzen des ECAP-Signals;
eine Fläche unter einer Kurve mindestens eines Abschnitts des ECAP-Signals;
eine Latenz mindestens eines Merkmals des ECAP-Signals; einen Spektralgehalt des ECAP-Signals;
ein Vorhandensein eines oder mehrerer Merkmale des ECAP-Signals;
ein Fehlen eines oder mehrerer Merkmale des ECAP-Signals oder
eine Kombination aus mindestens einer Spitze und mindestens einem Tal des ECAP-Signals.

7. System nach einem der Ansprüche 1 bis 6, wobei die Verarbeitungsschaltungsanordnung (208) ferner so ausgelegt ist, dass sie, basierend auf dem ECAP-Signal, mindestens eines von Folgenden einstellt:

einen Verstärkungswert, der zumindest teilweise die Einstellung des Parameters bestimmt;
eine oder mehrere Filtercharakteristiken des ECAP-Signals oder
eine Sensorelektrodenkombination, die zum Erfassen des ECAP-Signals verwendet wird.

8. System nach einem der Ansprüche 1 bis 7, wobei die erste Folge elektrischer Stimulationsimpulse zwei oder mehr Impulsfolgen umfasst, die eine mittlere Frequenz aufweisen, die kleiner als die erste Frequenz und größer als die zweite Frequenz ist.

9. System nach einem des Anspruchs 8, wobei die mittlere Frequenz aus einem Frequenzbereich von 150 Hz bis 900 Hz ausgewählt ist.

10. System nach einem der Ansprüche 1 bis 9, wobei das erste Zielgewebe einen ersten Zelltyp umfasst und das zweite Zielgewebe einen zweiten Zelltyp umfasst, der sich von dem ersten Zelltyp unterscheidet.

11. System nach einem der Ansprüche 1 bis 10, wobei die Verarbeitungsschaltungsanordnung (208) so ausgelegt ist, dass sie die Verabreichung der ersten Folge und die Verabreichung der zweiten Folge steuert, indem sie zumindest die Verabreichung der ersten Folge und der zweiten Folge elektrischer Stimulationsimpulse in einer wiederholbaren Reihe von Schlitzen steuert, wobei die wiederholbare Reihe von Schlitzen für die Verabreichung der ersten Folge elektrischer Stimulationsimpulse und der zweiten Folge elektrischer Stimulationsimpulse im Zeitablauf wiederholbar ist, und wobei:

die Verabreichung der ersten Folge elektrischer Stimulationsimpulse ein Erzeugen eines Impulses für einen ersten Schlitz zumindest einiger der wiederholbaren Reihen von Schlitzen umfasst, der die erste Frequenz erreicht, und
die Verabreichung der zweiten Folge elektrischer Stimulationsimpulse ein Erzeugen eines Impulses für einen zweiten Schlitz zumindest einiger der wiederholbaren Reihe von Schlitzen umfasst, der die zweite Frequenz erreicht.

12. System nach einem der Ansprüche 1 bis 11, wobei die zweite Frequenz aus einem Frequenzbereich von 40 Hz bis 60 Hz ausgewählt ist.

13. System nach einem der Ansprüche 1 bis 12, wobei der Parameter eine Amplitude umfasst und wobei der erste Wert der Amplitude, der zumindest teilweise die erste Folge elektrischer Stimulationsimpulse definiert, unter mindestens einer von einer Wahrnehmungsschwelle oder einer sensorischen Schwelle eines Patienten liegt.

14. System nach einem der Ansprüche 1 bis 13, ferner umfassend eine implantierbare medizinische Vorrichtung, die die Verarbeitungsschaltungsanordnung (208) umfasst.

15. System nach einem der Ansprüche 1 bis 14, wobei das erste Zielgewebe Gliazellen umfasst und wobei das zweite Zielgewebe Neuronen umfasst.

**Revendications**

1. Système comprenant :
   un circuit de traitement (208) configuré pour :

   commander l'administration d'un premier train d'impulsions de stimulation électrique à une première fréquence à un premier tissu cible ;
   commander l'administration d'un second train d'impulsions de stimulation électrique à une seconde fréquence à un second tissu cible différent du premier tissu cible, au moins certaines impulsions de stimulation électrique du premier train d'impulsions de stimulation électrique étant entrelacées avec au moins certaines impulsions de stimulation électrique du second train d'impulsions de stimulation électrique, et la première fréquence étant supérieure à la seconde fréquence ;
   recevoir un signal de potentiel d'action composé évoqué (ECAP) déclenché par une impulsion du second train d'impulsions de stimulation électrique ;
   ajuster, sur la base du signal ECAP, une première valeur d'un paramètre qui définit au moins partiellement le premier train d'impulsions de stimulation électrique à une seconde valeur ; et
   en réponse à l'ajustement de la première valeur du paramètre à la seconde valeur, commander l'administration des impulsions suivantes du premier train d'impulsions de stimulation électrique en fonction de la seconde valeur du paramètre.

2. Système selon la revendication 1, comprenant en outre un circuit de génération de stimulation configuré pour administrer le premier train d'impulsions de stimulation électrique et le second train d'impulsions de stimulation électrique, et le circuit de traitement (208) étant configuré pour commander le circuit de génération de stimulation (204) afin d'administrer le premier train d'impulsions de stimulation électrique et le second train d'impulsions de stimulation électrique.

3. Système selon l'une quelconque des revendications 1 et 2, le paramètre comprenant l'un des éléments suivants :

   une combinaison d'électrodes du premier train d'impulsions de stimulation électrique ;
   un nombre d'impulsions dans le premier train au cours d'un cycle de travail ;
   la première fréquence des impulsions du premier train ;
   une amplitude d'impulsions dans le premier train ;
   une largeur d'impulsion des impulsions du premier train ; un facteur de modulation de fréquence qui module la première fréquence du premier train ;
   un facteur de modulation d'amplitude qui module l'amplitude des impulsions du premier train ;
   un intervalle d'interphase des impulsions du premier train ;
   une forme d'impulsion des impulsions du premier train ; ou
   une polarité des électrodes du premier train.

4. Système selon l'une quelconque des revendications 1 à 3, comprenant en outre un circuit de détection configuré pour détecter le signal ECAP déclenché par l'impulsion du second train d'impulsions de stimulation électrique.

5. Système selon la revendication 4, le circuit de traitement (208) étant configuré pour déterminer, à partir du signal ECAP, une valeur caractéristique qui est une amplitude ECAP d'une partie du signal ECAP, le paramètre comprenant une première amplitude de train d'impulsions du premier train, les impulsions du second train comprenant une seconde amplitude de train, et le circuit de traitement (208) étant en outre configuré pour ajuster la première valeur du paramètre à la seconde valeur du paramètre par au moins :

   la soustraction de l'amplitude ECAP d'une valeur cible d'amplitude ECAP pour le patient afin de générer une amplitude différentielle ;
   la multiplication de l'amplitude différentielle par une valeur de gain pour générer une valeur différentielle préliminaire ;
   la multiplication de la valeur différentielle préliminaire par un facteur d'échelle pour générer une valeur différentielle informée, le facteur d'échelle représentant le rapport ;
   l'ajout de la valeur différentielle informée à la première valeur de la première amplitude de train pour générer la seconde valeur de la première amplitude de train ; et
   l'ajout de la valeur différentielle préliminaire à la première valeur de la seconde amplitude de train pour générer une seconde valeur de la seconde amplitude de train pour les impulsions suivantes du second train.

6. Système selon l'une quelconque des revendications 1 à 5, le circuit de traitement (208) :

déterminant une valeur caractéristique du signal ECAP ; et
ajustant, sur la base de la valeur caractéristique, la première valeur du paramètre qui définit au moins partiellement le premier train d'impulsions de stimulation électrique à la seconde valeur,
la valeur caractéristique comprenant :

une amplitude ECAP entre deux pics du signal ECAP ;
une aire sous une courbe d'au moins une partie du signal ECAP ;
une latence d'au moins une caractéristique du signal ECAP ;
un contenu spectral du signal ECAP ;
une présence d'une ou de plusieurs caractéristiques du signal ECAP ;
une absence d'une ou plusieurs caractéristiques du signal ECAP ; ou
une combinaison d'au moins un pic et d'au moins un creux du signal ECAP.

7. Système selon l'une quelconque des revendications 1 à 6, le circuit de traitement (208) étant en outre configuré pour ajuster, sur la base du signal ECAP, au moins l'un des éléments suivants :

une valeur de gain qui détermine au moins partiellement l'ajustement du paramètre ;
une ou plusieurs caractéristiques de filtrage du signal ECAP ; ou
une combinaison d'électrodes de détection utilisée pour détecter le signal ECAP.

8. Système selon l'une quelconque des revendications 1 à 7, le premier train d'impulsions de stimulation électrique comprenant deux trains d'impulsions ou plus qui ont une fréquence moyenne inférieure à la première fréquence et supérieure à la seconde fréquence.

9. Système selon l'une quelconque des revendications 8, la fréquence moyenne étant choisie dans une plage de fréquences allant de 150 Hz à 900 Hz.

10. Système selon l'une quelconque des revendications 1 à 9, le premier tissu cible comprenant un premier type de cellules et le second tissu cible comprenant un second type de cellules différent du premier type de cellules.

11. Système selon l'une quelconque des revendications 1 à 10, le circuit de traitement (208) étant configuré pour commander l'administration du premier train et l'administration du second train en commandant au moins l'administration du premier train et du second train d'impulsions de stimulation électrique dans une série répétable de créneaux, la série répétable de créneaux étant répétée dans le temps pour l'administration du premier train d'impulsions de stimulation électrique et du second train d'impulsions de stimulation électrique, et :

l'administration du premier train d'impulsions de stimulation électrique comprenant la génération d'une impulsion pour un premier créneau d'au moins une partie de la série de créneaux répétables qui atteint la première fréquence, et
l'administration du second train d'impulsions de stimulation électrique comprenant la génération d'une impulsion pour un second créneau d'au moins une partie de la série de créneaux répétables qui atteint la seconde fréquence.

12. Système selon l'une quelconque des revendications 1 à 11, la seconde fréquence étant choisie dans une plage de fréquences allant de 40 Hz à 60 Hz.

13. Système selon l'une quelconque des revendications 1 à 12, le paramètre comprenant une amplitude, et la première valeur de l'amplitude qui définit au moins partiellement le premier train d'impulsions de stimulation électrique étant inférieure à au moins un seuil de perception ou un seuil sensoriel d'un patient.

14. Système selon l'une quelconque des revendications 1 à 13, comprenant en outre un dispositif médical implantable comprenant le circuit de traitement (208).

15. Système selon l'une quelconque des revendications 1 à 14, le premier tissu cible comprenant des cellules gliales, et le second tissu cible comprenant des neurones.

**100**

**102**

**106**

**108A** **108B**

**110**

EXTERNAL
PROGRAMMER
**104**

**FIG. 1**

FIG. 2

300

MEMORY
304

PROCESSING
CIRCUITRY
302

USER
INTERFACE
306

TELEMETRY
CIRCUITRY
308

POWER
SOURCE
310

FIG. 3

FIG. 4

**FIG. 5A**

**FIG. 5B**

FIG. 5C

EP 4 277 697 B1

FIG. 5D

600

602 — DELIVER INFORMED PULSE ACCORDING TO A PREDETERMINED PULSE FREQUENCY OVER A PERIOD OF TIME

604 — DELIVER CONTROL PULSE? — NO

YES

606 — DELIVER, OVER THE PERIOD OF TIME, A CONTROL PULSE INTERLEAVED WITH AT LEAST SOME THE INFORMED PULSES

608 — SENSE, AFTER CONTROL PULSE AND PRIOR TO AN IMMEDIATELY SUBSEQUENT INFORMED PULSE, A RESPECTIVE ECAP

610 — ADJUST, BASED ON THE ECAP, ONE OR MORE INFORMED PARAMETER VALUES THAT AT LEAST PARTIALLY DEFINE THE INFORMED PULSES

FIG. 6

FIG. 7

EP 4 277 697 B1

800

| 802 | SELECT TARGET ECAP AMPLITUDE |
| --- | --- |

| 804 | SUBTRACT MEASURED AMP FROM TARGET ECAP AMP TO GENERATE DIFFERENTIAL AMP |
| --- | --- |

| 808 | MULTIPLY DIFFERENTIAL AMP BY GAIN VALUE TO GENERATE PRELIM DIFFERENTIAL VALUE |
| --- | --- |

| 816 | MULTIPLY PRELIM DIFFERENTIAL VALUE BY SCALING FACTOR TO GENERATE NEW THERAPY DIFFERENTIAL VALUE |
| --- | --- |

| 810 | ADD PRELIM DIFFERENTIAL VALUE TO CONTROL PULSE AMP TO GENERATE CONTROL PULSE AMP |
| --- | --- |

| 818 | ADD THERAPY DIFFERENTIAL VALUE TO INFORMED PULSE AMP |
| --- | --- |

| 812 | DELIVER CONTROL PULSE DEFINED BY NEW CONTROL PULSE AMP AT SCHEDULED TIME |
| --- | --- |

| 820 | DELIVER INFORMED PULSE DEFINED BY ADJUSTED THERAPY PULSE AMP AT SCHEDULED TIME |
| --- | --- |

| 814 | MEASURE AMP OF SENSED ECAP ELICITED BY THE DELIVERED CONTROL PULSE |
| --- | --- |

FIG. 8

**EP 4 277 697 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019246582 A1

- WO 2019246579 A1